(19) **Europäisches Patentamt / European Patent Office / Office européen des brevets**

(11) **EP 4 671 751 A1**

(12) **EUROPEAN PATENT APPLICATION**
published in accordance with Art. 153(4) EPC

(43) Date of publication:
**31.12.2025 Bulletin 2026/01**

(21) Application number: **24760418.4**

(22) Date of filing: **21.02.2024**

(51) International Patent Classification (IPC):
**G01N 27/12** *(2006.01)*

(52) Cooperative Patent Classification (CPC):
**G01N 27/12**

(86) International application number:
**PCT/JP2024/006393**

(87) International publication number:
**WO 2024/177120 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.02.2023 JP 2023027831**

(71) Applicant: **Japan Science and Technology Agency**
**Kawaguchi-shi, Saitama 332-0012 (JP)**

(72) Inventors:
• **MAJIMA Yutaka**
**Tokyo 152-8550 (JP)**
• **SUN Yexiao**
**Tokyo 152-8550 (JP)**
• **YAMAGUCHI Wataru**
**Tokyo 152-8550 (JP)**
• **IZAWA Seiichiro**
**Tokyo 152-8550 (JP)**

(74) Representative: **Scott, Stephen John**
**YUJA IP LAW**
**East Coast House**
**25 Skeldergate**
**York YO1 6DH (GB)**

(54) **HYDROGEN GAS SENSOR AND PROCESS FOR PRODUCING SAME**

(57) A hydrogen gas sensor that has high sensitivity and excellent response/recovery characteristics, and can detect hydrogen gas with low power consumption is provided. A hydrogen gas sensor 100 according to this disclosure has a substrate 10 having an insulating surface, first and second pad electrodes 12A and 12B formed on the insulating surface of the substrate 10, and a nanowire 14. The nanowire 14 made of a hydrogen storage metal is formed on the insulating surface of the substrate 10 to connect the first and second pad electrodes 12A and 12B, and has a linewidth of 50 nm or more and 150 nm or less, and a thickness of 10 nm or more and 60 nm or less. The hydrogen gas sensor 100 detects hydrogen gas based on variations in an electric signal detected between the first and second pad electrodes 12A and 12B by passing a current between the first and second pad electrodes 12A and 12B.

FIG. 1A

FIG. 1B

# FIG. 1C

# FIG. 1D

**Description**

TECHNICAL FIELD

This disclosure relates to a hydrogen gas sensor using a nanowire made of a specific metal, and a method of producing the same.

BACKGROUND

Various types of hydrogen gas sensors have been developed as hydrogen gas sensors that are suitable for applications in detecting hydrogen gas leaking from various types of devices that handle the hydrogen gas, such as fuel cells, and in measuring the concentrations of hydrogen gas in devices that handle the hydrogen gas. Among these, hydrogen gas sensors are known in which a hydrogen gas detector is sandwiched between a pair of electrodes, and hydrogen gas is detected based on variations in current or resistance detected between the electrodes while a constant voltage is applied between the electrodes.

As such hydrogen gas sensors, for example, as described in non-patent literature (NPL) 1, a hydrogen gas sensor with a hydrogen gas detector of palladium (Pd), which is a hydrogen storage metal, in the form of a thin film is known.

CITATION LIST

Non-patent Literature

NPL 1: T. Xu and M. P. Zach, Self-assembled monolayer-enhanced hydrogen sensing with ultrathin palladium films, Appl. Phys. Lett. 86, 203104 (2005)

SUMMARY

(Technical Problem)

However, conventional hydrogen gas sensors, which include hydrogen gas sensors with Pd thin films, have room for improvement in terms of important characteristics in practical application, such as sensitivity, response/recovery speed, and power consumption.

Considering the above problem, this disclosure aims to provide a hydrogen gas sensor that has high sensitivity and excellent response/recovery characteristics, and that can detect hydrogen gas with low power consumption, as well as a suitable method of producing the same.

(Solution to Problem)

In order to solve the above problem, the inventors have carefully considered the matter and obtained the following findings. The inventors have conceived a hydrogen gas sensor using a nanowire made of a specific hydrogen storage metal such as palladium (Pd). It has been found that sensor characteristics such as sensitivity, response/recovery characteristics, and power consumption are improved by making a hydrogen gas detector in the form of a nanowire, not a thin film, of Pd or other hydrogen storage metals. In particular, it has been found that by setting the linewidth and thickness of the nanowire to specific ranges, the cross-sectional shape of the nanowire perpendicular to its extension direction is made appropriate, and as an estimation mechanism is described later, the occlusion and desorption of hydrogen in and from the nanowire occur easily, which significantly improves the above sensor characteristics.

The gist of this disclosure, which has been completed based on the above findings, is as follows.

[1] A hydrogen gas sensor including:

a substrate having an insulating surface;
a first pad electrode and a second pad electrode that are formed on the insulating surface of the substrate; and
a nanowire made of a hydrogen storage metal, the nanowire being formed on the insulating surface of the substrate to connect the first pad electrode and the second pad electrode, the nanowire having a linewidth of 50 nm or more and 150 nm or less, and a thickness of 10 nm or more and 60 nm or less,
wherein the hydrogen gas sensor is configured to detect hydrogen gas based on a variation in an electric signal detected between the first pad electrode and the second pad electrode by passing a current between the first pad electrode and the second pad electrode.

[2] The hydrogen gas sensor according to [1] above, wherein the nanowire has a linewidth of 80 nm or more and 100 nm or less.

[3] The hydrogen gas sensor according to [1] or [2] above, wherein the nanowire has a thickness of 20 nm or more and 50 nm or less.

[4] The hydrogen gas sensor according to any one of [1] to [3] above, wherein the nanowire has a length of 10 $\mu$m or more and 300 mm or less.

[5] The hydrogen gas sensor according to [4] above, wherein the nanowire has a length of 0.07 mm or more.

[6] The hydrogen gas sensor according to [5] above, wherein the nanowire has a length of 0.5 mm or more.

[7] The hydrogen gas sensor according to any one of [1] to [6] above, wherein the hydrogen storage metal is one or more selected from

(I) single elements of palladium (Pd), rhodium (Rh), iridium (Ir), ruthenium (Ru), osmium (Os), vanadium (V), titanium (Ti), zirconium (Zr), lanthanum (La), tungsten (W), calcium (Ca), magnesium (Mg), strontium (Sr), barium (Ba), and beryllium (Be), as exothermic metals A that easily form stable hydrides, and solid solution alloys of these exothermic alloys A; and

(II) $AB_5$-type alloys, $AB_2$-type alloys, AB-type alloys, and $A_2B$-type alloys that are combinations of the exothermic metal(s) A and one or more selected from nickel (Ni), iron (Fe), cobalt (Co), manganese (Mn), and zinc (Zn), as endothermic metals B that have no affinity for hydrogen.

[8] The hydrogen gas sensor according to any one of [1] to [6] above, wherein the hydrogen storage alloy is palladium (Pd).

[9] The hydrogen gas sensor according to [8] above, wherein the palladium constituting the nanowire is polycrystallized.

[10] The hydrogen gas sensor according to [9] above, wherein the palladium constituting the nanowire has a lattice constant of 3.925 $\pm$ 0.005 angstroms.

[11] A method of producing a hydrogen gas sensor, the method including:

preparing a substrate having an insulating surface;

forming a first pad electrode and a second pad electrode on the insulating surface of the substrate; and

forming a nanowire made of a hydrogen storage metal on the insulating surface of the substrate to connect the first pad electrode and the second pad electrode, the nanowire having a linewidth of 50 nm or more and 150 nm or less, and a thickness of 10 nm or more and 60 nm or less,

wherein the method produces a hydrogen gas sensor that is configured to detect hydrogen gas based on a variation in an electric signal detected between the first pad electrode and the second pad electrode by passing a current between the first pad electrode and the second pad electrode.

[12] The method of producing a hydrogen gas sensor according to [11] above, the method including:

exposing the nanowire to an atmosphere containing hydrogen and an inert gas; and

thereafter subjecting the nanowire to heat treatment under an atmosphere containing hydrogen and an inert gas.

[13] The method of producing a hydrogen gas sensor according to [12] above, wherein the heat treatment is RTA treatment that is carried out at a heat treatment temperature of 350°C or more and 650°C or less.

[14] The method of producing a hydrogen gas sensor according to any one of [11] to [13] above, wherein the hydrogen storage alloy is palladium (Pd).

(Advantageous Effect)

The hydrogen gas sensor according to this disclosure has high sensitivity and excellent response/recovery characteristics, and can detect hydrogen gas with low power consumption. In addition, the method of producing a hydrogen gas sensor according to this disclosure can produce a hydrogen gas sensor that has high sensitivity and excellent response/recovery characteristics, and that can detect hydrogen gas with low power consumption.

BRIEF DESCRIPTION OF THE DRAWINGS

In the accompanying drawings:

FIG. 1A is a schematic perspective view of a hydrogen gas sensor 100 according to an embodiment of this disclosure;

FIG. 1B is a schematic top plan view of the hydrogen gas sensor 100;

FIG. 1C is a cross-sectional view taken on the line I-I of FIG. 1B;

FIG. 1D is a cross-sectional view taken on the line II-II of FIG. 1B;

FIG. 2 is a circuit diagram illustrating an example of measurement of sensor characteristics of the hydrogen gas sensor 100 (resistance sharing voltage measurement);

FIG. 3 is a cross-sectional view of a nanowire 14 perpendicular to its extension direction, in the hydrogen gas sensor 100;

FIG. 4 is a top plan view illustrating an example of arrangement of the nanowire 14 when the nanowire 14 is long, in the hydrogen gas sensor 100 according to an embodiment of this disclosure;

FIGS. 5A to 5D are diagrams illustrating a process of producing the gas sensor 100 according to an embodiment of this disclosure;

FIG. 6 is cross-sectional SEM images of nanowires with various linewidths, perpendicular to their extension directions, in Experimental Example 1;

FIG. 7 is (upper) graphs illustrating variations in a current value over time and (lower) graphs illustrating variations in a resistance change rate over time, for various linewidths in Experimental Example 1;

FIG. 8 is a graph illustrating the relationship between linewidth and sensitivity (sensitivity based on resistance change rate) at various operating temperatures $T$ obtained in Experimental Example 1;

FIG. 9 is a graph illustrating the relationship between linewidth and response time $t_{res50}$ at various operating temperatures $T$ obtained in Experimental Example 1;

FIG. 10 is a graph illustrating the relationship between linewidth and recovery time $t_{rec50}$ at various operating temperatures $T$ obtained in Experimental Example 1;

FIG. 11 is a graph illustrating the relationship between linewidth and activation energy of response (hydrogen occlusion reaction) and recovery (hydrogen desorption reaction) obtained from the results illustrated in FIGS. 9 and 10 in Experimental Example 1;

FIG. 12 is an SEM image illustrating part of a Pd nanowire (a length of 7 mm) produced using electron beam lithography (EBL) in Experimental Example 2;

FIG. 13A is graphs illustrating variations in a current value over time for various wire lengths, in Experimental Example 2;

FIG. 13B is graphs illustrating variations in a current value over time for various wire lengths, in Experimental Example 2;

FIG. 14 is a graph illustrating variations in a resistance change rate over time for various wire lengths, in Experimental Example 2;

FIG. 15 is a (upper) graph illustrating the relationship between wire length and response time $t_{res50}$ and a (lower) graph illustrating the relationship between wire length and recovery time $t_{rec50}$ obtained in Experimental Example 2;

FIG. 16 is a graph illustrating variations in a voltage change rate over time at various hydrogen gas concentrations, in Experimental Example 3;

FIG. 17 is a graph illustrating variations in a voltage change rate over time at various applied voltages, in Experimental Example 4;

FIG. 18 is a graph illustrating variations in a voltage change rate over time at various operating temperatures, in Experimental Example 5;

FIG. 19 is graphs illustrating variations in a resistance value over time when carrier gas is dry air (upper graph) and when carrier gas is nitrogen (lower graph), in Experimental Example 6;

FIG. 20 is a graph illustrating variations in a resistance change rate over time when carrier gas is dry air and when carrier gas is nitrogen, in Experimental Example 6;

FIG. 21 is a graph illustrating variations in a resistance change rate over time for various wire thicknesses, in Experimental Example 7;

FIG. 22 is a graph illustrating variations in a resistance change rate over time for repeated introduction of hydrogen gas, for a wire thickness of 30 nm and a wire length of 31 mm, in Experimental Example 8;

FIG. 23 is a graph illustrating variations in a resistance change rate over time for repeated introduction of hydrogen gas, for a wire thickness of 10 nm and a wire length of 18 mm, in Experimental Example 8;

FIG. 24 is a cross-sectional SEM image (upper image) of a nanowire perpendicular to its extension direction, and an EDS elemental mapping (lower image) of the same area as this cross-sectional SEM image, in Experimental Example 9;

FIG. 25 is a (upper) graph illustrating variations in a resistance value over time and a (lower) graph illustrating variations in a resistance change rate over time, at an operating temperature of 24°C, in Experimental Example 9;

FIG. 26 is a (upper) graph illustrating variations in a resistance value over time and a (lower) graph illustrating variations in a resistance change rate over time, at an operating temperature of 50°C, in Experimental Example 9;

FIG. 27 is a (upper) graph illustrating variations in a resistance value over time and a (lower) graph illustrating variations in a resistance change rate over time, at an operating temperature of 100°C, in Experimental Example 9;

FIG. 28 is a (upper) graph illustrating variations in a resistance value over time and a (lower) graph illustrating variations in a resistance change rate over time, at an operating temperature of 150°C, in Experimental Example 9;

FIG. 29 is a (upper) graph illustrating variations in a resistance value over time and a (lower) graph illustrating variations in a resistance change rate over time, at an operating temperature of 21°C (room temperature), in Experimental Example 9;

FIG. 30 is a (upper) graph illustrating the relationship between operating temperature and response time $t_{res50}$ and a (lower) graph illustrating the relationship between operating temperature and recovery time $t_{rec50}$ obtained in Experimental Example 9;

FIG. 31 is a graph illustrating the relationship between operating temperature and sensitivity (sensitivity based on resistance change rate) obtained in Experimental Example 9;

FIG. 32 is a graph illustrating X-ray diffraction spectra obtained by GI-WAXS measurement on three conditions (Condition 1: No heat treatment, Condition 2: 5-minute annealing at 250°C, Condition 3: RTA treatment at 500°C) in Experimental Example 10;

FIG. 33 is SEM images of top surfaces of nanowires on three conditions (Condition 3: RTA treatment at 500°C, Condition 4: RTA treatment at 400°C, Condition 5: RTA treatment at 600°C) in Experimental Example 10;

FIG. 34 is a graph illustrating variations in a resistance change rate over time on Conditions 1 to 3 in Experimental Example 10;

FIG. 35 is a graph illustrating variations in a resistance change rate over time on Conditions 2 to 5 in Experimental Example 10; and

FIG. 36 is a graph illustrating variations in a resistance change rate over time on Condition 3 in Experimental Example 10.

DETAILED DESCRIPTION

(Hydrogen Gas Sensor)

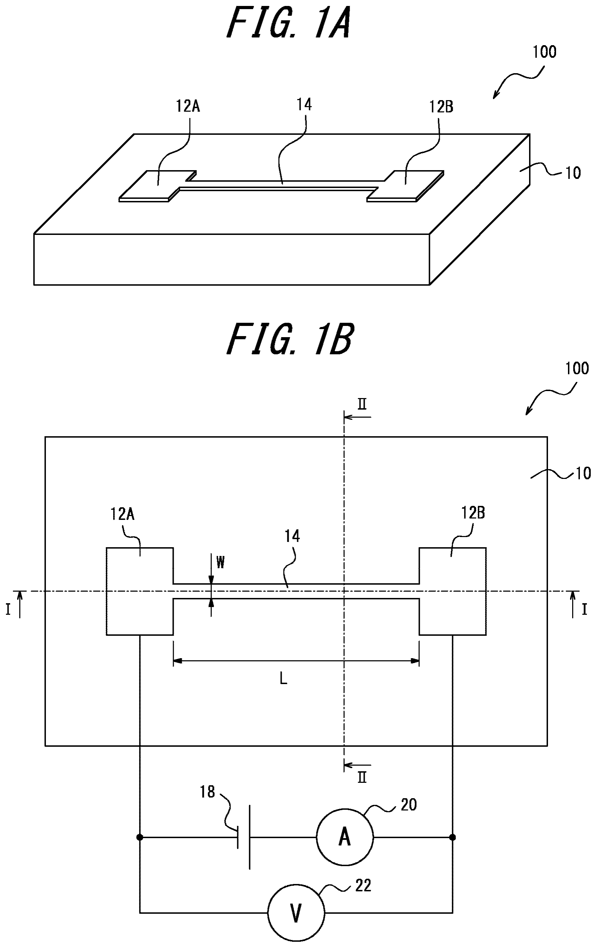

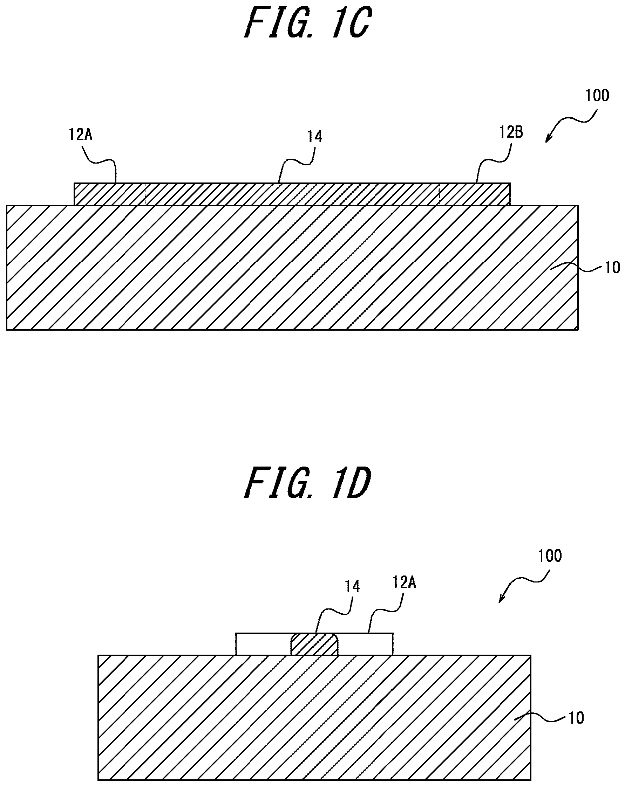

Referring to FIGS. 1A, 1B, 1C, and 1D, a hydrogen gas sensor 100 according to an embodiment of this disclosure has a substrate 10, a first pad electrode 12A and a second pad electrode 12B, and a nanowire 14 made of a specific hydrogen storage metal. The first pad electrode 12A and the second pad electrode 12B are formed on the substrate 10. The nanowire 14 is formed on the substrate 10 to connect the first pad electrode 12A and the second pad electrode 12B. Although detailed actions and effects are described below, this embodiment achieves improvement in sensor characteristics, such as sensitivity, response/recovery characteristics, and power consumption, due to the action of this nanowire 14.

[Mechanism of Hydrogen Gas Detection]

The hydrogen gas sensor 100 detects hydrogen gas based on variations in an electric signal detected between the first pad electrode 12A and the second pad electrode 12B by passing a current between the first pad electrode 12A and the second pad electrode 12B. For example, as illustrated in FIG. 1B, a power supply 18 and an ammeter 20 are connected in series between the first pad electrode 12A and the second pad electrode 12B, and a voltmeter 22 is connected in parallel with this. In this case, while a constant voltage, which can be measured by the voltmeter 22, is applied between the first pad electrode 12A and the second pad electrode 12B by the power supply 18, the ammeter 20 detects variations in current between the first pad electrode and the second pad electrode, to detect hydrogen gas based on the detected variations in current. Alternatively, while a constant current is passed between the first pad electrode 12A and the second pad electrode 12B, the gas can be detected based on variations in voltage detected between the first pad electrode 12A and the second pad electrode 12B. Alternatively, instead of the above variations in current or voltage, the gas can also be detected based on variations in resistance detected between the first pad electrode 12A and the second pad electrode 12B. In other words, the above "electric signal" refers to current, voltage, or resistance. In this embodiment, the circuit of the gas sensor can be configured with the two terminals, so the hydrogen gas sensor can be constructed without increasing wiring or circuitry.

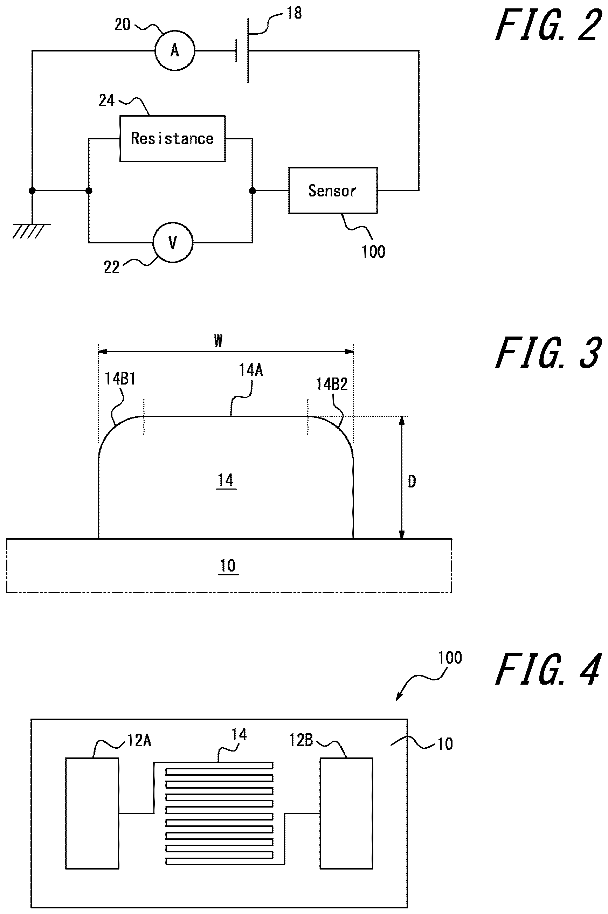

To measure the sensor characteristics, resistance sharing voltage measurement can be performed as illustrated in FIG. 2. In FIG. 2, a resistance 24 with a known resistance value is connected in series to the hydrogen gas sensor 100, the power supply 18, and the ammeter 20, and the voltmeter 22 is connected in parallel with this resistance 24. In this case, while a constant voltage is applied between the first pad electrode (not illustrated) and the second pad electrode (not illustrated) of the hydrogen gas sensor 100 by the power supply 18, the voltmeter 22 detects variations in voltage applied to the resistance 24, to detect hydrogen gas based on the detected variations in voltage. In this configuration, the voltmeter 22 indirectly detects the variations in voltage across the hydrogen gas sensor 100 (i.e., between the first pad electrode and the second pad electrode).

Furthermore, the voltmeter 22 that is connected in parallel with the resistance 24 in FIG. 2 may be changed to be connected in parallel with the hydrogen gas sensor 100. In this case, while a constant voltage is applied between the first pad electrode (not illustrated) and the second pad electrode (not illustrated) of the hydrogen gas sensor 100 by the power supply 18, the voltmeter 22 directly detects variations in voltage across the hydrogen gas sensor 100 (i.e., between the first pad electrode and the second pad electrode), to detect hydrogen gas based on the detected variations in voltage.

[Substrate]

The substrate 10 supports the first pad electrode 12A, the second pad electrode 12B, and the nanowire 14, which is a hydrogen gas detector. The substrate 10 is not particularly limited as long as the substrate 10 has an insulating surface, and any substrate, such as an insulating substrate e.g., a glass substrate, an alumina substrate, or a zirconia substrate, or a silicon substrate with a silicon oxide film or silicon nitride film formed on its surface, can be used. The shape and dimensions of the substrate 10 are not particularly limited, but when a substrate with a rectangular main surface is used, the dimensions can be in a range of, for example, length: 1 to 300 mm × width: 1 to 300 mm × thickness: 0.1 to 1.2 mm.

[First Pad Electrode and Second Pad Electrode]

The first pad electrode 12A and the second pad electrode 12B are a pair of electrodes that is necessary for supplying a current to the nanowire 14 and detecting variations in an electric signal corresponding to variations in the concentration of hydrogen gas. The shape and dimensions of the first pad electrode 12A and the second pad electrode 12B are not particularly limited, as long as the first pad electrode 12A and the second pad electrode 12B are formed on the insulating surface of the substrate 10, but when the shape of the main surface is rectangular, the dimensions can be in a range of, for example, length: 30 to 1000 $\mu$m × width: 30 to 1000 $\mu$m × thickness: 5 to 500 nm. The metal constituting the first pad electrode 12A and the second pad electrode 12B is not particularly limited, and can be any metal, but can be one or more selected from platinum, gold, and palladium, for example. From the perspective of process simplicity, the metal can be the same type of metal as the nanowire 14 described later.

[Nanowire]

The nanowire 14 is an element that constitutes a gas detector, and is formed to connect the first pad electrode 12A and the second pad electrode 12B on the insulating surface of the substrate 10. It is essential that the nanowire 14 is made of a hydrogen storage metal. The hydrogen storage metal is preferably one or more selected from single elements of palladium (Pd), rhodium (Rh), iridium (Ir), ruthenium (Ru), osmium (Os), vanadium (V), titanium (Ti), zirconium (Zr), lanthanum (La), tungsten (W), calcium (Ca), magnesium (Mg), strontium (Sr), barium (Ba), and beryllium (Be), as exothermic metals A that easily form stable hydrides, and solid solution alloys of these exothermic alloys A. The hydrogen storage metal is also preferably one or more selected from $AB_5$-type alloys, $AB_2$-type alloys, AB-type alloys, and $A_2B$-type alloys that are combinations of the exothermic metal(s) A and one or more selected from nickel (Ni), iron (Fe), cobalt (Co), manganese (Mn), and zinc (Zn), as endothermic metals B that have no affinity for hydrogen. The $AB_5$-type alloys include $LaNi_5$, $CaNi_5$, and the like. The $AB_2$-type alloys include $MaZn_2$, $ZrNi_2$, and the like. The AB-type alloys include TiFe, TiNi, and the like. The $A_2B$-type alloys include $Mg_2Ni$, $Ca_2Fe$, and the like. Among these, the hydrogen storage metal is more preferably composed of palladium (Pd). The hydrogen gas sensor 100 therefore responds (detects hydrogen gas) due to the occlusion of hydrogen in the nanowire 14, and the hydrogen gas sensor 100 recovers (detects the off of hydrogen gas) due to the desorption of hydrogen from the nanowire 14.

[Width of Nanowire]

Referring to FIGS. 1A to 1D and FIG. 3, it is important that the linewidth W of the nanowire 14 is 50 nm or more and 150 nm or less. This has the remarkable effects of achieving high sensitivity and excellent response/recovery characteristics. The inventors consider that the following actions are responsible for such effects. As illustrated in FIG. 3, the nanowire 14 has curved surface portions 14B1 and 14B2 at both ends in its width direction, and a top surface that connects the pair of curved surface portions 14B1 and 14B2 is a flat portion 14A. The internal stress $\Delta P$ applied to the nanowire 14 is expressed by the following Equation (1).

$$\Delta P = \gamma \left( 1/R1 + 1/R2 \right) \qquad \cdots (1)$$

Here, $\gamma$ is the surface tension of a metal constituting the nanowire 14, R1 is the radius of curvature of the nanowire 14 in the width direction, and R2 is the radius of curvature of the nanowire 14 in its extension direction. Since the nanowire 14 has no curvature in its extension direction, R2 is infinite, and the term of 1/R2 in Equation (1) can be considered as zero. Then, the internal stress ΔP becomes larger as the radius of curvature R1 in the width direction becomes smaller. As illustrated in FIG. 3, the cross-sectional shape of the nanowire 14 perpendicular to its extension direction is a so-called dome shape (semicylindrical), so the internal stress is distributed in a cross-sectional lateral direction (width direction). For example, the surface tension of a clean surface of palladium is approximately 1000 mN/m. When the radius of curvature R1 of the curved surface portions 14B1 and 14B2 of the nanowire 14 is set to 5 to 30 nm, for example, the internal stress applied to the curved surface portions 14B1 and 14B2 of the nanowire 14 is tentatively calculated as about 200 to 30 MPa. Although the clean surface of palladium cannot be maintained on the nanowire, palladium has a strong catalytic effect, so there is a surface that adsorbs and desorbs hydrogen. However, in the actual nanowire, the surface tension of a surface with the radius of curvature is almost two orders of magnitude smaller than the above estimate, so the internal stress is approximately 0.6 to 1 MPa. Here, in the PCT (P: pressure, C: hydrogen storage capacity, T: temperature) characteristic curves for general hydrogen storage metals, the range of hydrogen equilibrium pressure of 0.6 to 1 MPa is a region in which variations in hydrogen equilibrium pressure with respect to variations in hydrogen storage capacity are small at room temperature, for both occlusion (exothermic reaction) and desorption (endothermic reaction). For this reason, it is easy for hydrogen to be occluded in and desorbed from the curved surface portions 14B1 and 14B2 of the nanowire 14 with an internal stress of about 0.6 to 1 MPa. In contrast, the flat portion 14A (especially its middle portion) of the nanowire 14 is in a state equivalent to a thin film, so no internal stress is applied thereto, but only atmospheric pressure (approximately 0.1 MPa) is applied thereto. In the PCT (P: pressure, C: hydrogen storage capacity, T: temperature) characteristic curves of general hydrogen storage metals, the range of hydrogen equilibrium pressure around 0.1 MPa is a region in which variations in hydrogen equilibrium pressure with respect to variations in hydrogen storage capacity are large at room temperature (a region in which hydrogen storage capacity does not vary much with respect to variations in hydrogen equilibrium pressure), for both occlusion (exothermic reaction) and desorption (endothermic reaction). For this reason, it is difficult for hydrogen to be occluded in and desorbed from the flat portion 14A (especially its middle portion) of the nanowire 14. Using the nanowire 14 as the hydrogen detector allows an increase in the ratio of the curved surface portions to the flat portion, compared to a thin film. This contributes to improving the sensor characteristics.

From another perspective, even when there is no occlusion of hydrogen at all, the internal stress has already been applied to the curved surface portions 14B1 and 14B2 of the nanowire 14 as if a certain amount of hydrogen were occluded there due to their shape. In other words, the amount of hydrogen itself that can be occluded in the curved surface portions 14B1 and 14B2 is less than that in the thin film or the flat portion 14A. Therefore, it is thought that, in the curved surface portions 14B1 and 14B2, hydrogen is occluded in areas into and from which hydrogen can easily enter and exit.

The occlusion process of hydrogen in palladium has the steps of [1] $H_2$ adsorption, [2] dissociation of $H_2$ into atomic hydrogen 2H, and [3] diffusion of atomic hydrogen within palladium. The desorption process of hydrogen from palladium has the steps of [A] atomic hydrogen occluded in palladium diffuses in a surface, [B] atomic hydrogen forms a covalent bond to become $H_2$, and [C] $H_2$ is desorbed from the surface. In both occlusion and desorption, the rate-determining step is the diffusion of atomic hydrogen. Here, when the internal stress is high, the diffusion of atomic hydrogen in palladium is suppressed. As a result, it is thought that the diffusion of atomic hydrogen is suppressed in the curved surface portions 14B1 and 14B2, and that hydrogen is occluded in areas into and from which hydrogen can easily enter and exit.

Now, the topic is returned to the linewidth W of the nanowire 14. When the linewidth W is 50 nm or more and 150 nm or less, it is thought that the balance between the curved surface portions 14B1 and 14B2 and the flat portion 14A is optimal, and the occlusion and desorption of hydrogen in and from the nanowire 14 occur easily, which remarkably improves the sensor characteristics. When the linewidth W is less than 50 nm, the ratio of the curved surface portions 14B1 and 14B2, which have high internal stress, to the flat portion 14 becomes too large. As a result, it becomes difficult for hydrogen to be occluded in the nanowire 14, which causes insufficiency of sensitivity. When the linewidth W exceeds 150 nm, the ratio of the flat portion 14A, to which only atmospheric pressure is applied, to the curved surface portions 14B1 and 14B2 becomes too large. In this case, the state is close to a thin film, so atomic hydrogen diffuses easily in the nanowire 14, and hydrogen is occluded in areas into and from which hydrogen cannot easily enter and exit, thus causing difficulty in the occlusion and desorption of hydrogen. Therefore, the response/recovery characteristics are not sufficiently obtained. When the linewidth W is 50 nm or more and 150 nm or less, the balance between the curved surface portions 14B1 and 14B2 and the flat portion 14A is optimal, and the internal stress is applied to the curved surface portions 14B1 and 14B2 and their vicinity. In this case, while the diffusion of atomic hydrogen is suppressed, a small amount of hydrogen is occluded in areas into and from which hydrogen can easily enter and exit, so the occlusion and desorption of hydrogen in and from the nanowire 14 occur easily, which significantly improves the sensor characteristics. From this perspective, the linewidth W should be 50 nm or more, and preferably 80 nm or more. In addition, the linewidth W should be 150 nm or less, and preferably 100 nm or less.

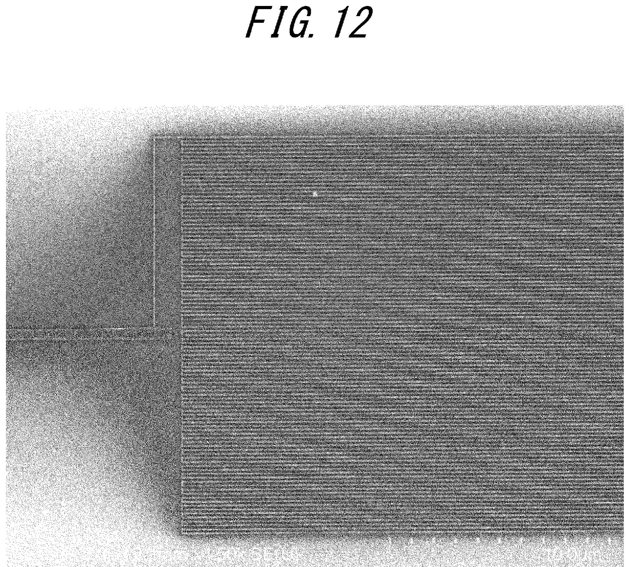

In this specification, the linewidth W of the nanowire 14 means the width of the nanowire 14 at the lowest point (interface with the substrate 10), as illustrated in FIG. 3. As the linewidth W of the nanowire 14, the average value of linewidths obtained in SEM images, in which the cross-sections of the nanowire 14 perpendicular to its extension direction are observed using a SEM at 10 points at equal intervals (e.g., intervals of 0.1 to 100 $\mu$m) in the extension direction, is adopted. In the arrangement of the nanowire as illustrated in FIGS. 4 and 12, cross-sections at 10 points at equal intervals can be observed in a single SEM image.

[Thickness of Nanowire]

Referring to FIG. 3, it is important that the thickness D of the nanowire 14 is 10 nm or more and 60 nm or less. When the thickness D is less than 10 nm, the curved surface portions become narrow due to the thin thickness, and the volume of palladium decreases, which results in a decrease in sensitivity and failure to obtain good response/recovery characteristics. Therefore, the thickness D should be 10 nm or more, and preferably 20 nm or more. On the other hand, when the thickness D exceeds 60 nm, the diffusion length of atomic hydrogen in a thickness direction becomes long, which results in failure to obtain good response/recovery characteristics. Therefore, the thickness D should be 60 nm or less, and preferably 50 nm or less.

In this specification, the thickness D of the nanowire 14 means the distance from the lowest point (interface with the substrate 10) to the highest point (flat portion 14A) of the nanowire 14, as illustrated in FIG. 3. As the thickness D of the nanowire 14, the average value of thicknesses obtained in SEM images, in which the cross-sections of the nanowire 14 perpendicular to its extension direction are observed using a SEM at 10 points at equal intervals (e.g., intervals of 0.1 to 100 $\mu$m) in the extension direction, is adopted. In the arrangement of the nanowire as illustrated in FIGS. 4 and 12, cross-sections at 10 points at equal intervals can be observed in a single SEM image.

[Length of Nanowire]

Referring to FIG. 1B, the length L of the nanowire 14 is not particularly limited, but from the perspective of further improving the response/recovery characteristics and sensitivity to hydrogen gas, the length L of the nanowire 14 is preferably 10 $\mu$m or more. In order to obtain good response/recovery characteristics even when an operating temperature is at room temperature, the length L is more preferably 0.07 mm or more, and even more preferably 0.5 mm or more. The upper limit of the length L is not particularly limited, as a longer length is preferable from the perspective of sensor characteristics. However, the length L is preferably 300 mm or less due to constraints on production process and from the perspective of the size of the sensor. In addition, one of the features of the hydrogen gas sensor 100 according to this embodiment is that the length L of the nanowire 14 is long. When making the nanowire 14 longer, the nanowire 14 can be arranged in a zigzag pattern on the substrate 10, as illustrated in FIG. 4, to reduce the size of the sensor. In this specification, the length L of the nanowire 14 refers to the length of a single nanowire that connects the first pad electrode 12A and the second pad electrode 12B. The length L of the nanowire 14 can be measured by observing the top surface of the hydrogen gas sensor 100 using a SEM.

[Crystal State of Hydrogen Storage Alloy Constituting Nanowire]

For example, in the nanowire 14 immediately after being formed by electron beam evaporation or sputtering (As deposition), the crystal state of the hydrogen storage alloy is a mixture of amorphous and polycrystal. Although details are described in a production method and Experimental Example 10 below, when the hydrogen storage alloy constituting the nanowire 14 is palladium, the crystallization of palladium progresses and palladium is polycrystallized by subjecting the nanowire 14 to heat treatment while palladium is occluding hydrogen. The polycrystallization of palladium constituting the nanowire is preferable because the response/recovery characteristics are more improved. Note that, the "polycrystallization of palladium" means that the crystallization of palladium progresses and amorphous disappears, and the crystal state of palladium is polycrystallized. The polycrystallization of palladium can be determined based on the intensity and area of a peak attributed to Pd (111) in an X-ray diffraction spectrum obtained by Grazing Incidence Wide Angle X-ray Scattering (GI-WAXS) measurement. Both the peak intensity and the peak area significantly increase in the polycrystalline state after the heat treatment, compared to the state of a mixture of amorphous before the heat treatment.

As described in the production method and Experimental Example 10 below, it has been found that when the heat treatment performed on palladium that has occluded hydrogen is Rapid Thermal Anneal (RTA) treatment, the crystallization progresses through the heat treatment with the lattice constant of palladium increased, and the increased lattice constant is maintained even after the heat treatment. Furthermore, it has been found that using the nanowire 14 made of palladium with such an increased lattice constant improves the response/recovery characteristics more significantly.

The $\alpha$-phase of palladium is a hydrogen solid solution phase (Pd+H) that exists when x < 0.02 as PdHx (x: the ratio of atomicity of H to Pd). In the $\alpha$-phase, hydrogen atoms are randomly dissolved in the lattice, and the lattice constant of the $\alpha$-phase is 3.90 $\pm$ 0.02 angstroms. In contrast, the $\beta$-phase of palladium is a hydride phase (Pd-H) that exists when x > 0.6. In the $\beta$-phase, hydrogen atoms occupy the octahedral sites (O-sites) in the lattice, and the lattice constant of the $\beta$-phase is 4.02 $\pm$ 0.02 angstroms. In other words, the lattice constant of the $\beta$-phase is 2 to 3% larger than that of the $\alpha$-phase. Note

that, there is a coexistence region (0.02 ≤ x ≤ 0.6) of the α-phase and the β-phase near room temperature. According to the research of the inventors, it has been found that when palladium that has occluded hydrogen is subjected to the RTA treatment, the lattice constant of palladium constituting the nanowire 14 becomes 3.925 ± 0.005 angstroms. In other words, although it cannot be said that the palladium constituting the nanowire 14 has transformed to the β-phase, the palladium has reached a value close to the upper limit of the lattice constant of the α-phase.

The inventors consider that the mechanism behind the more significant improvement in the response/recovery characteristics in this case is as follows. When palladium that has occluded hydrogen is subjected to the RTA treatment, crystallization progresses through the heat treatment with the increased lattice constant of palladium. Here, to the nanowire 14, large internal stress is applied due to its radius of curvature. Therefore, even after the heat treatment (i.e., in the absence of hydrogen), the lattice constant is maintained at an increased state due to the large internal stress. For example, in the case of palladium in a thin film, even when the lattice constant increases due to the occlusion of hydrogen, the lattice constant returns to its original state once the hydrogen is no longer present. Considering this, in this disclosure, the fact that the lattice expansion state is maintained even in the absence of a hydrogen atmosphere after the heat treatment, due to the large internal stress applied to the nanowire 14, is a unique phenomenon. In this lattice expansion state, atomic hydrogen can easily enter and exit, so it is thought that the response/recovery characteristics are improved significantly.

The lattice constant of palladium constituting the nanowire 14 can be calculated from each of a peak attributed to Pd (111) and a peak attributed to Pd (200) in an X-ray diffraction spectrum obtained by GI-WAXS measurement, and it has been confirmed that the both lattice constants are the same.

[Effects]

As described above, the hydrogen gas sensor 100 according to this embodiment has high sensitivity and excellent response/recovery characteristics, and can detect hydrogen gas with low power consumption. The hydrogen gas sensor 100 according to this embodiment also has the effect of being able to detect hydrogen gas even at low operating temperatures (for example, even at room temperature). In addition, the hydrogen gas sensor 100 according to this embodiment has the effect of being able to detect hydrogen gas even at low hydrogen gas concentrations, and therefore has a wide range of detectable hydrogen gas concentrations.

(Method of Producing Hydrogen Gas Sensor)

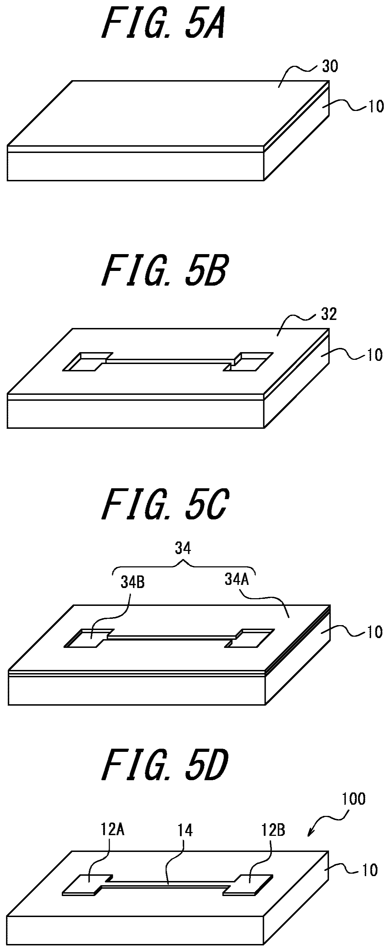

Referring to FIGS. 5A to 5D, a method of producing a hydrogen gas sensor 100 according to an embodiment of this disclosure will be described. The method of producing the hydrogen gas sensor 100 according to this embodiment includes the step of preparing a substrate 10, the step of forming a first pad electrode 12A and a second pad electrode 12B, and the step of forming a nanowire 14. The hydrogen gas sensor 100 according to this embodiment as described above can be produced by this method.

First, a substrate 10 is prepared. The details of the substrate 10 are as described above. Next, an example of the steps of forming a first pad electrode 12A, a second pad electrode 12B, and a nanowire 14 will be described.

Referring to FIG. 5A, a resist film 30 is formed on the substrate 10. The resist film 30 can be formed by applying a resist composition for electron beam exposure onto the substrate 10 and drying the resist composition. The application method is not particularly limited, but spin coating can be used suitably. The thickness of the resist film 30 can be set as appropriate so as to be thicker than the thickness of the nanowire 14 to be formed (or thicker than both the nanowire 14 and the pad electrodes 12A and 12B when the nanowire 14 and the pad electrodes 12A and 12B are formed together). After the spin coating, the resist film 30 may be annealed under appropriate conditions to efficiently volatilize a solvent and increase the density of the resist film 30.

Next, as illustrated in FIG. 5B, the resist film 30 is developed to form a mask pattern 32 of a predetermined shape. The mask pattern 32 is formed by exposing and developing the resist film 30 by electron beam lithography. The shape and dimensions of an exposed part of the substrate 10, due to removal of the resist film by the mask pattern 32, correspond to the shape and dimensions (linewidth W and length L) of the nanowire 14 to be formed. Here, the shape of a concave portion of the mask pattern 32 can be adjusted by appropriately controlling the type of the resist composition, the thickness of the resist film 30 to be formed, and the dose of an electron beam to be applied. This allows adjustment of the shape and size (i.e., the radius of curvature, length in a width direction, length in a height direction, and the like) of curved surface portions 14B1, 14B2 of the nanowire 14 at both ends in a width direction. As illustrated in FIGS. 5B to 5D, when the nanowire 14 and the pad electrodes 12A and 12B are formed together, the shape of the exposed part of the substrate 10, due to removal of the resist film by the mask pattern 32, corresponds to the shape and dimensions of the nanowire 14 and the pad electrodes 12A and 12B to be formed.

Next, as illustrated in FIG. 5C, a metal film 34 is formed by electron beam evaporation or sputtering, for example. At this time, a first portion 34A of the metal film is formed on the mask pattern 32, and a second portion 34B of the metal film

EP 4 671 751 A1

is formed on the substrate 10, which is exposed by removal of the resist film by the mask pattern 32. Note that, the metal film 34 preferably includes a Ti layer, Cr layer, or Ta layer (adhesive layer) with a thickness of about 1 to 5 nm, and a layer made of a hydrogen storage metal (e.g., Pd), which constitutes the nanowire 14 and the pad electrodes 12A, 12B, formed thereon. Note that, the Ti layer, Cr layer, or Ta layer functions as an adhesive layer that bonds, onto the substrate 10, the layer made of the metal constituting the nanowire 14 and the pad electrodes 12A, 12B.

Next, as illustrated in FIG. 5D, the nanowire 14 and the pad electrodes 12A, 12B are formed on the substrate 10 by a lift-off process, in which the mask pattern 32 is removed to remove the first portion 34A of the metal film formed thereon. In this example, as illustrated in FIGS. 5B to 5D, the nanowire 14 and the pad electrodes 12A and 12B are formed together. However, only the nanowire 14 may be formed in the above process, and the pad electrodes 12A and 12B may be formed separately afterwards by general photolithography, for example. Note that, the Ti, layer, Cr layer, or Ta layer is formed as an adhesive layer to adhere the nanowire 14 onto the substrate 10, but during the process of evaporation of the metal constituting the nanowire 14, Ti, Cr, or Ta diffuses into the metal layer, and most of the Ti layer, Cr layer, or Ta layer disappears. Alternatively, the adhesive layer is almost impossible to see in a SEM image because the film thickness of the adhesive layer is very thin. Between the substrate 10 and the nanowire 14, Ti, Cr, or Ta remains in some places like islands, but in most places, the substrate 10 and the nanowire 14 are in direct contact.

After this, as an optional step, the nanowire 14 may be subjected to heat treatment. The heat treatment changes the cross-sectional shape of the nanowire 14 perpendicular to its extension direction to increase the radius of curvature, and makes the shape of the nanowire with a so-called "circular segment" cross-section, in which part of a circle is missing. As a result, internal stress is applied to a wide range of the nanowire, which achieves further improvement in sensor characteristics. Conditions for the heat treatment are preferably a heat treatment temperature (the maximum temperature of the atmosphere during the heat treatment): 250 to 400°C, and holding time at the heat treatment temperature: 1 to 90 minutes, in an inert gas atmosphere such as Ar containing hydrogen.

In addition, as optional steps, the step of exposing the nanowire 14 to an atmosphere containing hydrogen and an inert gas, and then the step of subjecting the nanowire 14 to heat treatment in an atmosphere containing hydrogen and an inert gas are preferably performed. This allows, when the hydrogen storage alloy constituting the nanowire is palladium, palladium to be polycrystallized, thus further improving response/recovery characteristics, as described above.

[Exposure Step]

The exposure step is carried out by exposing the nanowire 14 to an atmosphere containing hydrogen, an inert gas as a remaining part, and any unavoidable impurity gas that may be contained. This step allows palladium constituting the nanowire 14 to occlude hydrogen. The hydrogen content is preferably 1 to 5 volume%, and the inert gas as the remaining part can be one or more selected from argon (Ar), helium (He), and neon (Ne). From the perspective of ensuring that palladium occludes a sufficient amount of hydrogen, exposure time is preferably set to one minute or more, and from the perspective of saturation of the amount of hydrogen occluded by palladium, the exposure time is preferably set to ten minutes or less.

[Heat Treatment Step]

The heat treatment step can be carried out following the exposure step at atmospheric pressure in an atmosphere containing hydrogen, an inert gas as a remaining part, and any unavoidable impurity gas that may be contained. This heat treatment step promotes the crystallization of palladium. The hydrogen content is preferably set to 1 to 5 volume%, and the inert gas as the remaining part can be one or more selected from argon (Ar), helium (He), and neon (Ne).

The heat treatment step may be carried out using a general heat treatment furnace, but is preferably carried out by RTA treatment using an RTA device. As described above, RTA treatment causes crystallization to progress with the lattice constant of palladium increased, and the increased lattice constant is maintained even after the heat treatment. From the perspective of obtaining this effect sufficiently, a heat treatment temperature (the maximum temperature of the atmosphere during the heat treatment) is preferably 350°C or more and 650°C or less. The heat treatment temperature is more preferably 400°C or more. In addition, the heat treatment temperature is more preferably 600°C or less. From the perspective of obtaining the above effect sufficiently, a temperature increase rate from a temperature at the start of the heat treatment to the heat treatment temperature is 2°C/sec or more and 200°C/sec or less. Holding at the heat treatment temperature is not essential. Holding time at the heat treatment temperature can be set to 0 min or more and 10 min or less.

The gas sensor 100 can be produced through the above steps.

EXAMPLES

[Experimental Example 1: Effect of Linewidth W]

11

<Production of Hydrogen Gas Sensors>

Hydrogen gas sensors were produced using the following procedure. First, Si substrates (length: 15 mm × width: 15 mm × thickness: 0.6 mm) with surface layers of approximately 1 $\mu$m made of $SiO_2$ were prepared.

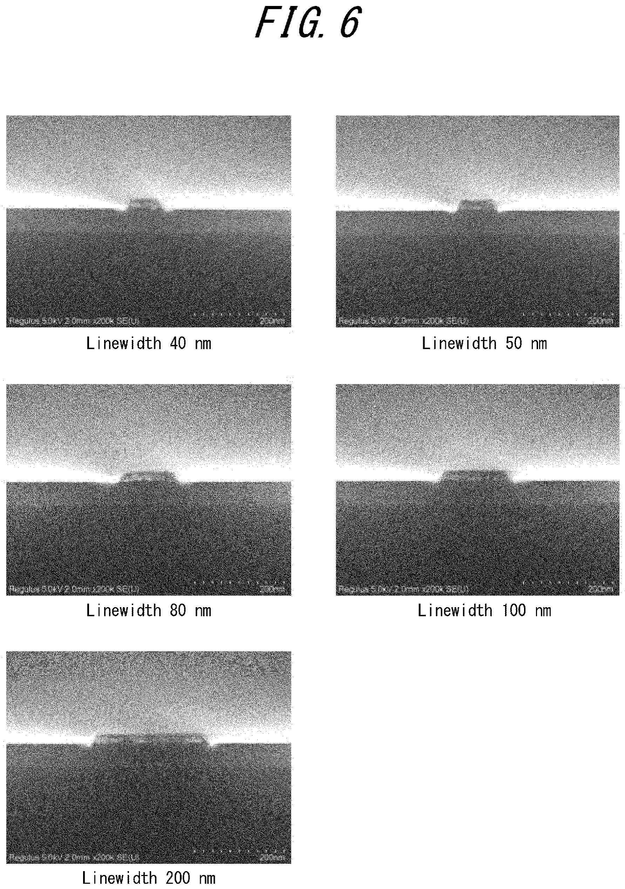

Pd nanowires were formed on the substrates using an electron beam lithography (EBL) device (Elionix, ELS-7500EX). Specifically, an electron beam resist ZEP-520A was applied to the substrates by spin coating to form resist films. Then, mask patterns of predetermined shapes were drawn using the EBL device. Then, Ti layers (thickness: 3 nm) and Pd layers (thickness: 30 nm), which were on the Ti layers, were formed by electron beam evaporation. Through a lift-off process that removes the mask patterns thereafter, the Pd nanowires (five conditions of linewidth W: 40 nm (comparison example), 50 nm (inventive example), 80 nm (inventive example), 100 nm (inventive example), and 200 nm (comparison example), wire thickness D: 30 nm, wire length L: 10 $\mu$m) were formed on the substrates. FIG. 6 illustrates cross-sectional SEM images of the nanowires with various widths, perpendicular to their extension directions. Note that, the Ti layer is formed as an adhesive layer to bond the Pd nanowire to the substrate, but because the Ti layer is very thin, most of the Ti layer disappears as Ti diffuses into the Pd layer in the process of evaporation of the Pd layer. Although Ti remains in some places like islands between the substrate and the Pd nanowire, the substrate and the Pd nanowire are in direct contact in most places.

Next, first pad electrodes and second pad electrodes (75 $\mu$m × 75 $\mu$m) each of which was constituted of a Ti layer (thickness: 5 nm) and a Pt layer (thickness: 40 nm), which was on the Ti layer, were produced by general photolithography. Thereby, the hydrogen gas sensors using the Pd nanowires were produced.

<Hydrogen Gas Detection Tests>

The above hydrogen gas sensors were placed in a measurement chamber in which a hydrogen concentration could be controlled using Ar + 3% $H_2$ gas, and tests were conducted using a semiconductor parameter analyzer system (Keysight Technologies B1500A) to detect hydrogen gas based on variations in current detected between the first pad electrode and the second pad electrode, while a constant voltage (V = 50 mV) was applied between the first pad electrode and the second pad electrode, and various sensor characteristics were evaluated. A circuit diagram is illustrated in FIG. 1B. The operating temperature T was set to 50°C. The measurement was carried out at atmospheric pressure, with $N_2$ gas (flow rate: 1 SLM), as a carrier gas, introduced into the measurement chamber, with a hydrogen gas concentration of 1% (10000 ppm) when hydrogen gas was ON. The hydrogen gas was switched ON and OFF three times. In other words, the hydrogen gas was switched ON at 0 seconds, OFF at 300 seconds, ON at 900 seconds, OFF at 1200 seconds, ON at 1800 seconds, and OFF at 2100 seconds, and the measurement was carried out until 3000 seconds.

<Dependence of Response/Recovery Characteristics on Linewidth>

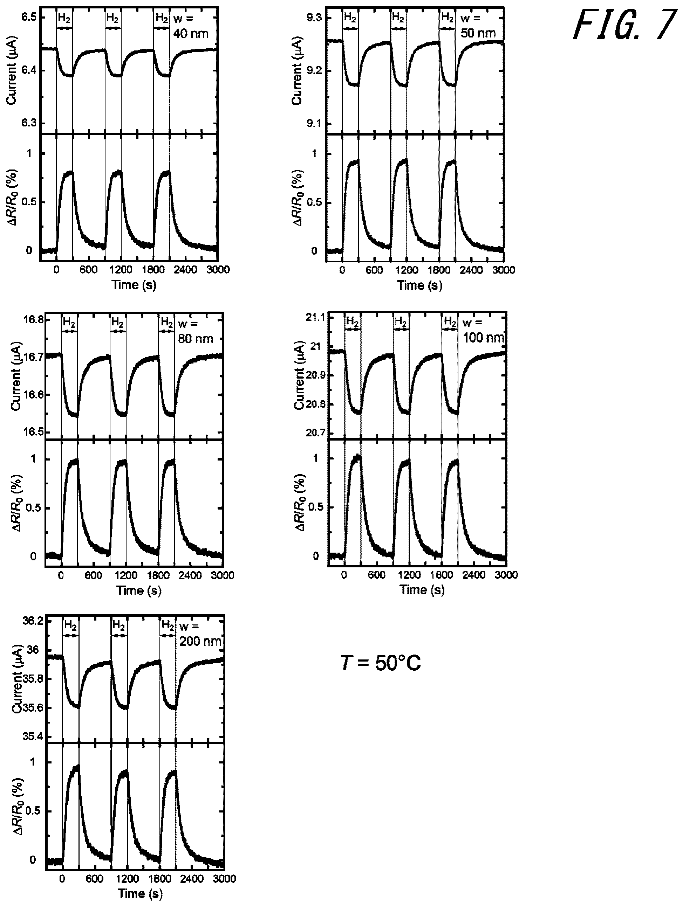

FIG. 7 (upper graphs) illustrates variations in a current value over time for various linewidths. FIG. 7 (lower graphs) illustrates variations in a resistance change rate over time, by converting the current value into a resistance value. Here, with respect to $\Delta R/R_0$ on the vertical axis, $R_0$ is a resistance value at 0 seconds, and $\Delta R$ is the difference between a resistance value at a certain time and $R_0$. For every linewidth, a decrease in current (an increase in resistance) was observed when hydrogen was ON, and an increase in current (a decrease in resistance) was observed when hydrogen was OFF. This is thought that when hydrogen is ON, scattering in electron conduction increases due to volume expansion caused by the diffusion of atomic hydrogen into palladium, and this causes the increase in resistance.

<Dependence of Sensitivity on Linewidth at Various Operating Temperatures>

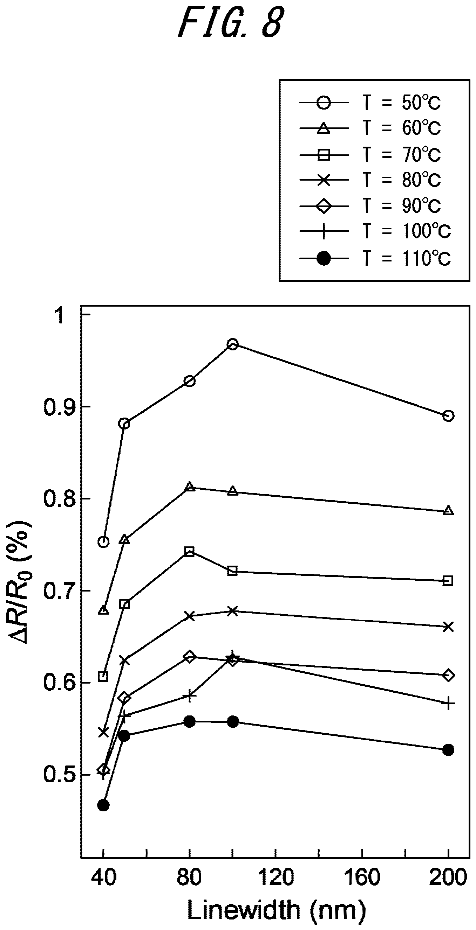

The same tests as described above were conducted at operating temperatures T of 60°C, 70°C, 80°C, 90°C, 100°C, and 110°C. FIG. 8 illustrates the relationship between linewidth and sensitivity (sensitivity based on resistance change rate) at various operating temperatures T. The sensitivity $\Delta R/R_0$ on the vertical axis of FIG. 8 is the average value of $\Delta R/R_0$ at the three OFF times (300 seconds, 1200 seconds, and 2100 seconds) when $R_0$ is fixed as a resistance value at 0 seconds.

From FIG. 8, the sensitivity was maximized in a range of a linewidth W of 80 to 100 nm.

When the linewidth W is less than 50 nm, the cross-sectional area of the nanowire perpendicular to its extension direction decreases, so the sensitivity decreases rapidly. In particular, when the linewidth W is 40 nm, the ratio of curved surface portions, which have high internal stress, to a flat portion of the nanowire becomes too large, and the internal stress is applied to the entire nanowire, so it becomes difficult for atomic hydrogen to enter the nanowire, which decreases the sensitivity rapidly.

When the linewidth W is 200 nm, the sensitivity decreases slightly, even though the cross-sectional area of the nanowire perpendicular to its extension direction increases, compared to when the linewidth W is 100 nm. The inventors think that this is due to the following mechanism. The increase in cross-sectional area is due to an increase in a flat portion of the nanowire, and only atmospheric pressure, not internal stress, is applied to the flat portion. Therefore, the flat portion is in a state equivalent to a thin film, so the sensitivity decreases slightly.

<Dependence of Response Time $t_{res50}$ on Linewidth at Various Operating Temperatures>

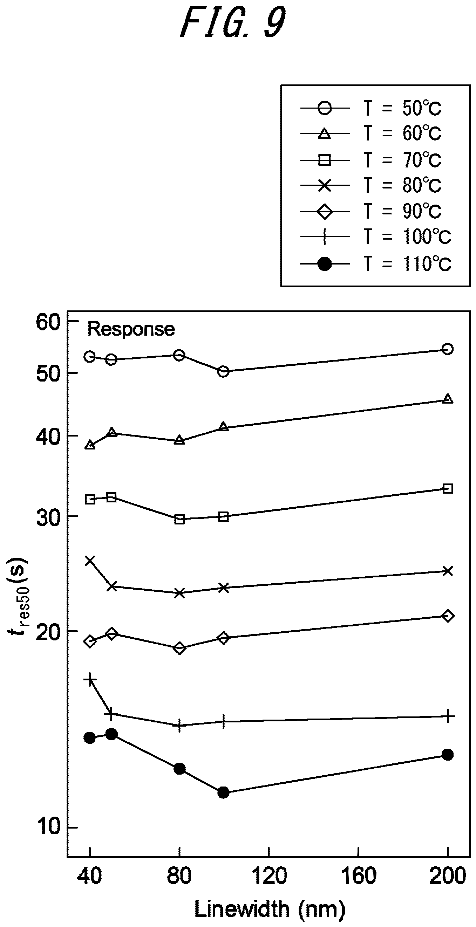

FIG. 9 illustrates the relationship between linewidth and response time $t_{res50}$ at various operating temperatures T. The response time $t_{res50}$ is the time it takes for a current value to vary from "a current value when hydrogen gas is ON" to 50% of "the current value when hydrogen gas is ON - a current value when hydrogen gas is OFF", and the shorter the time, the faster the response. Note that, the vertical axis of FIG. 9 represents the average value of $t_{res50}$ at the three ON/OFF times.

From FIG. 9, the response time $t_{res50}$ was the shortest in a range of a linewidth W of 80 to 100 nm.

When the linewidth W is less than 50 nm, the linewidth becomes close to the thickness of the nanowire, so while a flat portion decreases in the cross-sectional shape of the nanowire, the ratio of curved surface portions with a small radius of curvature increases. This causes an increase in the ratio of a cross-sectional area to which internal stress is applied, compared to when the linewidth is wider. The large internal stress at these curved surface portions is applied to the entire nanowire, and due to the internal stress close to the hydrogen equilibrium pressure at the time of absorbing hydrogen, it is difficult for hydrogen to be occluded, which results in longer response time.

When the linewidth W is 200 nm, a flat portion of the nanowire increases, and because internal stress caused by the shape of the nanowire is not applied to the flat portion, hydrogen continues to diffuse and to be occluded in a deep direction of the flat portion, which results in longer response time.

The higher the operating temperature, the shorter the response time. When the operating temperature is high, the hydrogen equilibrium pressure for hydrogen occlusion increases, which makes it difficult for hydrogen to be occluded. As only a small amount of hydrogen can enter, the response time becomes shorter. This is consistent with the fact that the higher the operating temperature, the lower the sensitivity.

<Dependence of Recovery Time $t_{rec50}$ on Linewidth at Various Operating Temperatures>

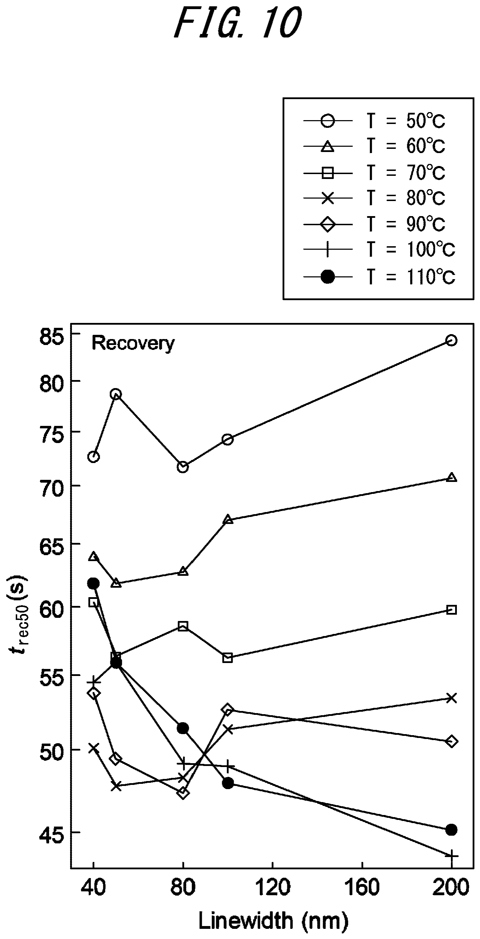

FIG. 10 illustrates the relationship between linewidth and recovery time $t_{rec50}$ at various operating temperatures T. The recovery time $t_{rec50}$ is the time it takes for a current value to vary from "a current value when hydrogen gas is OFF" to 50% of "a current when hydrogen gas is ON next time - the current when hydrogen gas is OFF", and the shorter the time, the faster the recovery. Note that, the vertical axis of FIG. 10 represents the average value of $t_{rcc50}$ at the three ON/OFF times.

From FIG. 10, the recovery time $t_{rec50}$ was the shortest in a range of a linewidth W of 50 to 80 nm.

When the linewidth W is less than 50 nm, the linewidth becomes close to the thickness of the nanowire, so while a flat portion decreases in the cross-sectional shape of the nanowire, the ratio of curved surface portions with a small radius of curvature increases. This causes an increase in the ratio of a cross-sectional area to which internal stress is applied, compared to when the linewidth is wider. The large internal stress at these curved surface portions is applied to the entire nanowire, and due to the internal stress close to the hydrogen equilibrium pressure at the time of releasing hydrogen, it is difficult for hydrogen to be desorbed, which results in longer recovery time.

At operating temperatures of 80°C or less, the recovery time became longer when the linewidth W exceeded 80 nm. This is thought to be because the ratio of a hydrogen storage capacity in a flat portion of the nanowire increases as the linewidth increases, so hydrogen is occluded in a deeper part of the flat portion, which causes difficulty in recovering. Conversely, this suggests that the recovery characteristic is better for the nanowire structure than for the flat film structure.

On the other hand, at operating temperatures of 90°C or more, the recovery time became shorter when the linewidth W exceeded 80 nm. This is thought to be because the hydrogen equilibrium pressure at the time of absorption increases with an increase in temperature, and a flat portion of the nanowire has a low hydrogen storage capacity, which makes it easier for hydrogen to be desorbed.

For linewidths W of 100 nm or more, the recovery time became shorter when the operating temperature was high. This is thought to be because, at high temperatures, the hydrogen equilibrium pressure for hydrogen desorption increases, which makes it easier for hydrogen to be desorbed.

In addition, when the linewidth W was 80 nm or less and the operating temperature was 100°C or more, the recovery time increased as the linewidth decreased. In particular, when the linewidth was less than 50 nm, it is difficult for hydrogen to be occluded due to internal stress caused by the shape, and it is difficult for hydrogen that has been even occluded to be desorbed. It is expected that the internal stress caused by the shape is greater than an increase in the hydrogen equilibrium pressure for desorption due to an increase in temperature.

<Dependence of Activation Energy for Response/Recovery on Linewidth>

The activation energy for response/recovery was determined using an Arrhenius plot.

First, from the results in FIG. 9, for each linewidth, 1000/T (K$^{-1}$) and Log(1/t$_{res50}$) were plotted on the horizontal axis and the vertical axis, respectively, and the slope of a straight line of the plots obtained by the method of least squares was determined. From this slope, the activation energy Ea for response (hydrogen occlusion reaction) was determined.

Similarly, from the results in FIG. 10, for each linewidth, 1000/T (K$^{-1}$) and Log(l/t$_{rec50}$) were plotted on the horizontal axis and the vertical axis, respectively, and the slope of a straight line of the plots obtained by the method of least squares was determined. From this slope, the activation energy Ea for recovery (hydrogen desorption reaction) was determined.

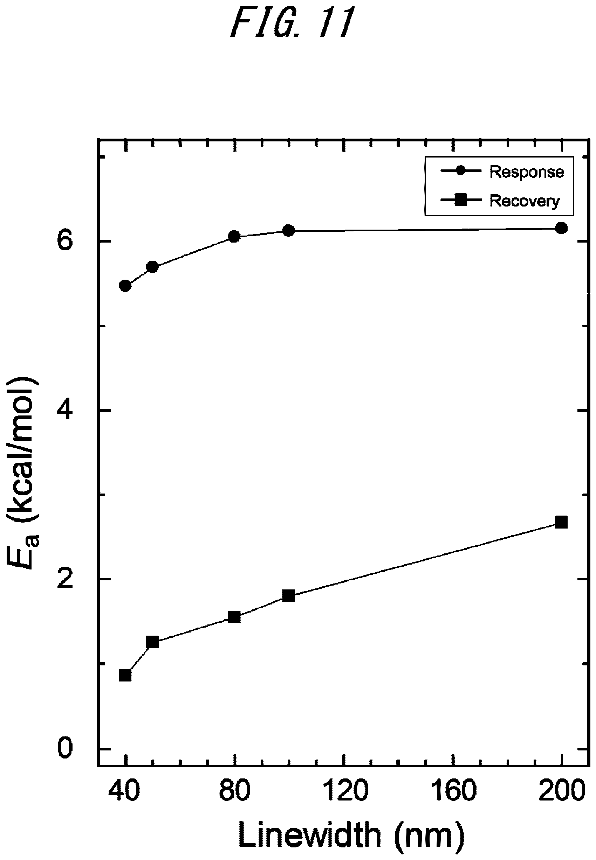

FIG. 11 illustrates the relationship between linewidth and the activation energy for response (hydrogen occlusion reaction) and recovery (hydrogen desorption reaction) obtained from the results illustrated in FIGS. 9 and 10. The activation energy for response increased as the linewidth W increased up to 100 nm, and then became constant when the linewidth W exceeded 100 nm. The activation energy for recovery increased as the linewidth W increased. In addition, the activation energy for response was about 3 to 4 kcal/mol greater than the activation energy for recovery. This is thought to be because the narrower the linewidth, the more difficult it is for hydrogen to be occluded due to internal stress caused by the shape, the smaller the storage capacity, the smaller the activation energy for hydrogen that can be occluded.

[Experimental Example 2: Effect of Wire Length L]

Hydrogen gas sensors were produced in the same manner as in Experimental Example 1, with a linewidth W fixed at 80 nm and a wire thickness D fixed at 30 nm, on five conditions of a line length L of 0.07 mm, 0.7 mm, 7 mm, 14 mm, and 21 mm. In this experimental example, in order to increase the length L of the wire, the nanowires were arranged as illustrated in FIG. 4 on four conditions of a wire length of 0.7 mm or more. The length of each row of the wire was 0.07 mm (70 $\mu$m), and the nanowire was arranged in an area of 70 $\mu$m × 70 $\mu$m for the maximum wire length. As a representative example, FIG. 12 illustrates a SEM image of part of the Pd nanowire (length: 7 mm).

Hydrogen gas detection tests were carried out in the same manner as in Experimental Example 1. However, in this experimental example, resistance sharing voltage measurement was carried out by adopting the circuit diagram illustrated in FIG. 2. The operating temperature T was set to room temperature 24°C (297 K). The applied voltage V was set to 1 V. The measurement was carried out at atmospheric pressure, with N$_2$ gas (flow rate: 1 SLM), as a carrier gas, introduced into a measurement chamber, with a hydrogen gas concentration of 1% (10000 ppm) when hydrogen gas was ON. The hydrogen gas was switched ON at 100 seconds and OFF at 700 seconds, and the measurement was carried out until 1300 seconds.

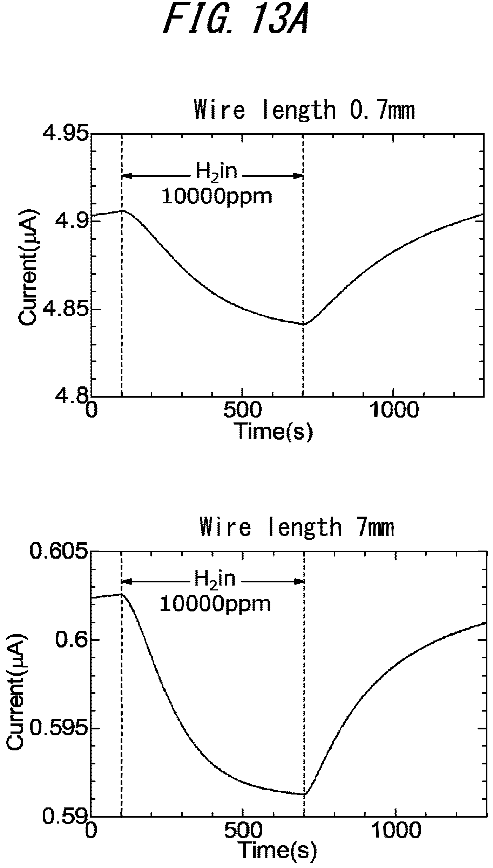

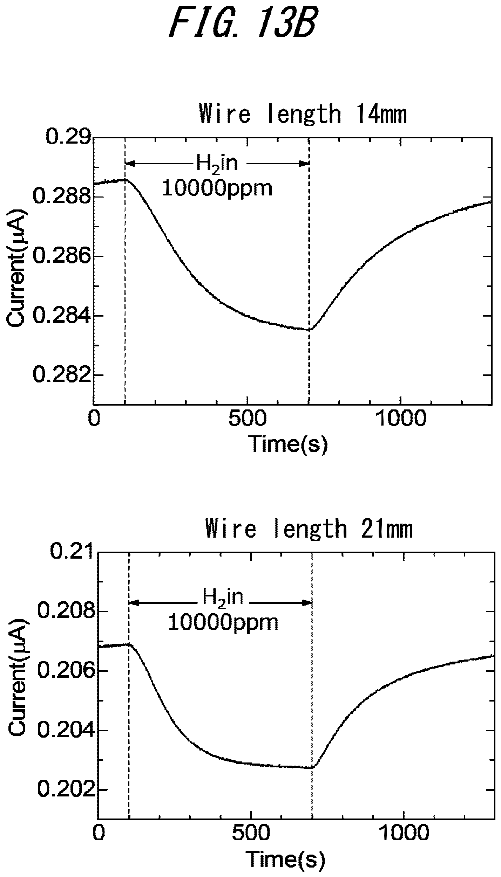

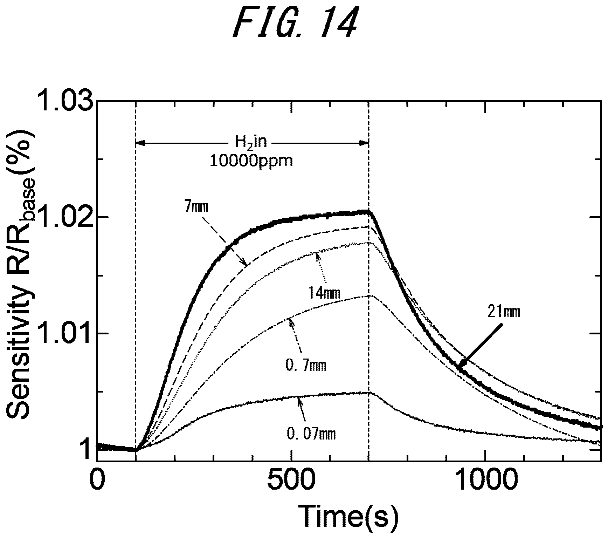

FIGS. 13A and 13B are graphs illustrating variations in a current value over time for various wire lengths, in which the current value was converted from a measurement value of voltage applied to a resistance portion. FIG. 14 illustrates variations in a resistance change rate over time, by converting the current value into a resistance value. Here, with respect to R/R$_{base}$ on the vertical axis, R$_{base}$ is a resistance value at 100 seconds (immediately before the hydrogen gas is ON), and R is a resistance value at a certain time. From FIG. 14, it is found that the longer the wire length, the higher the sensitivity (sensitivity based on the resistance change rate). This experimental example was conducted with the operating temperature T set to room temperature. The response/recovery characteristics at room temperature could be observed by increasing the wire length.

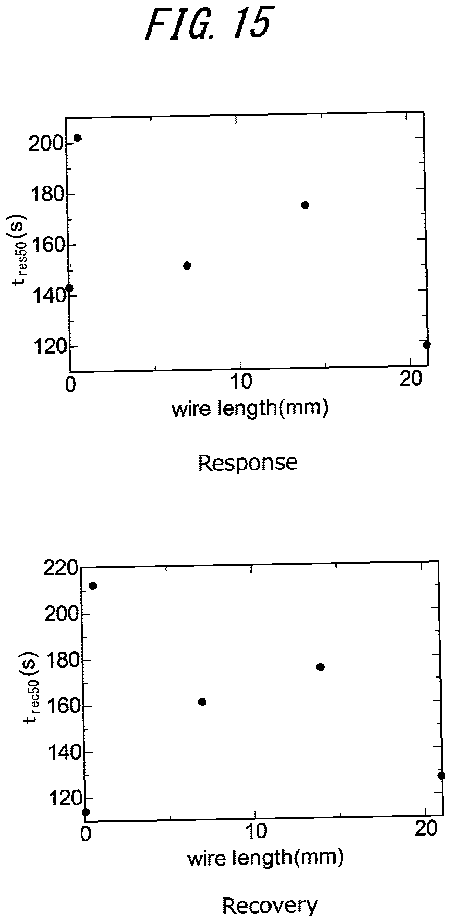

FIG. 15 is a (upper) graph illustrating the relationship between wire length and response time t$_{res50}$, and a (lower) graph illustrating the relationship between wire length and recovery time t$_{rec50}$. For both the response time t$_{res50}$ and the recovery time t$_{rec50}$, an overall trend in which the longer the wire length, the shorter the time can be seen.

[Experimental Example 3: Effect of Hydrogen Concentration]

A hydrogen gas sensor was produced in the same manner as in Experimental Example 1, with a linewidth W of 80 nm, a wire thickness D of 30 nm, and a wire length L of 14 mm. In this experimental example, a nanowire was arranged as illustrated in FIG. 4 because the wire length L was long.

Hydrogen gas detection tests were carried out in the same manner as in Experimental Example 1. However, in this experimental example, resistance sharing voltage measurement was carried out by adopting the circuit diagram illustrated in FIG. 2. The operating temperature T was set to room temperature 21°C (294 K). The applied voltage V was set to 1 V. The measurement was carried out at atmospheric pressure, with N$_2$ gas (flow rate: 1 SLM), as a carrier gas, introduced into a measurement chamber, on four conditions of a hydrogen gas concentration of 10000 ppm (1%), 6000 ppm (0.6%), 1400 ppm (0.14%), and 300 ppm (0.03%) when hydrogen gas was ON. The hydrogen gas was switched ON at 100 seconds and OFF at 400 seconds, and the measurement was carried out until 700 seconds.

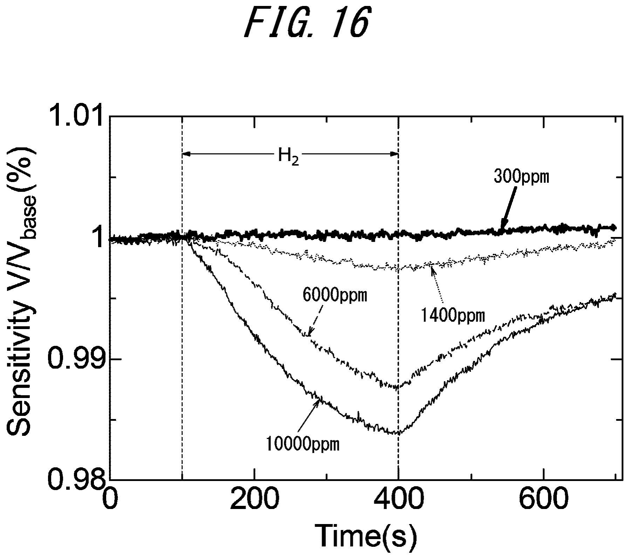

FIG. 16 is a graph illustrating variations in a voltage change rate over time at various hydrogen gas concentrations. Here, with respect to $V/V_{base}$ on the vertical axis, $V_{base}$ is a voltage value at 100 seconds (immediately before the hydrogen gas is ON), and V is a voltage value at a certain time. It was seen from FIG. 16 that the higher the hydrogen concentration, the higher the sensitivity (sensitivity based on the voltage change rate), and the sufficient sensitivity was obtained at hydrogen concentrations of 1400 ppm or more.

[Experimental Example 4: Effect of Applied Voltage]

A hydrogen gas sensor was produced in the same manner as in Experimental Example 1, with a linewidth W of 80 nm, a wire thickness D of 30 nm, and a wire length L of 14 mm. In this experimental example, a nanowire was arranged as illustrated in FIG. 4 because the wire length L was long.

Hydrogen gas detection tests were carried out in the same manner as in Experimental Example 1. However, in this experimental example, resistance sharing voltage measurement was carried out by adopting the circuit diagram illustrated in FIG. 2. The operating temperature T was set to room temperature 21°C (294K). The applied voltage V was set on four conditions of 10 V, 1 V, 0.5 V, and 0.1 V. The measurement was carried out at atmospheric pressure, with $N_2$ gas (flow rate: 1 SLM), as a carrier gas, introduced into a measurement chamber, with a hydrogen gas concentration of 1400 ppm (0.14%) when hydrogen gas was ON. The hydrogen gas was switched ON at 100 seconds and OFF at 400 seconds, and the measurement was carried out until 700 seconds.

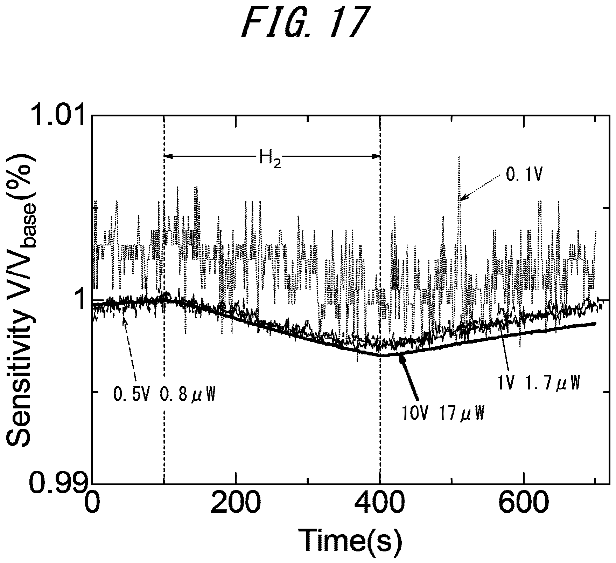

FIG. 17 is a graph illustrating variations in a voltage change rate over time at various applied voltages. Here, with respect to $V/V_{base}$ on the vertical axis, $V_{base}$ is a voltage value at 100 seconds (immediately before the hydrogen gas is ON), and V is a voltage value at a certain time. It was seen from FIG. 17 that the higher the applied voltage, the higher the sensitivity (sensitivity based on the voltage change rate), and the sufficient sensitivity was obtained at applied voltages of 0.5 V or more. The power consumption corresponding to an applied voltage of 0.5 V is 0.8 $\mu$W. In other words, the hydrogen gas sensor in this experimental example can operate at extremely low power consumption of the order of $\mu$W.

[Experimental Example 5: Effect of Operating Temperature]

A hydrogen gas sensor was produced in the same manner as in Experimental Example 1, with a linewidth W of 80 nm, a wire thickness D of 30 nm, and a wire length L of 14 mm. In this experimental example, a nanowire was arranged as illustrated in FIG. 4 because the wire length L was long.

Hydrogen gas detection tests were carried out in the same manner as in Experimental Example 1. However, in this experimental example, resistance sharing voltage measurement was carried out by adopting the circuit diagram illustrated in FIG. 2. The operating temperature T was set on four conditions of room temperature 21°C (294 K), 50°C (323 K), 75°C (348 K), and 100°C (373 K). The applied voltage V was set to 1 V. The measurement was carried out at atmospheric pressure, with $N_2$ gas (flow rate: 1 SLM), as a carrier gas, introduced into a measurement chamber, with a hydrogen gas concentration of 6000 ppm (0.6%) when hydrogen gas was ON. The hydrogen gas was switched ON at 100 seconds and OFF at 400 seconds, and the measurement was carried out until 700 seconds.

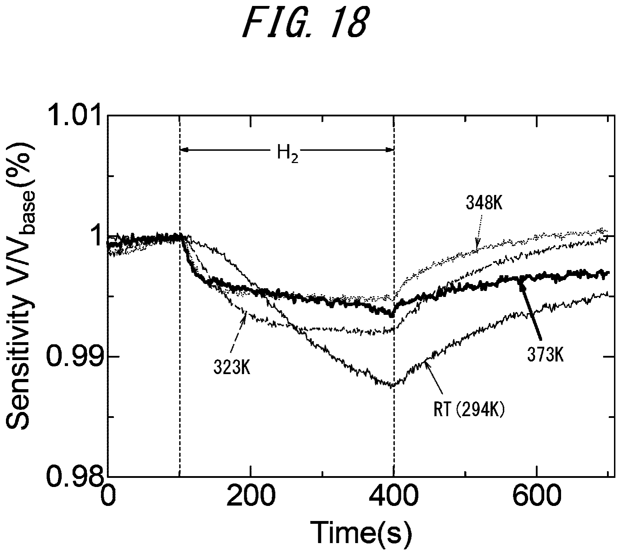

FIG. 18 is a graph illustrating variations in a voltage change rate over time at various operating temperatures. Here, with respect to $V/V_{base}$ on the vertical axis, $V_{base}$ is a voltage value at 100 seconds (immediately before the hydrogen gas is ON), and V is a voltage value at a certain time. It is seen from FIG. 18 that the hydrogen gas sensor in this experimental example can operate at room temperature.

[Experimental Example 6: Effect of Carrier Gas]

A hydrogen gas sensor was produced in the same manner as in Experimental Example 1, with a linewidth W of 80 nm, a wire thickness D of 30 nm, and a wire length L of 7 mm. In this experimental example, a nanowire was arranged as illustrated in FIG. 4 because the wire length L was long.

Hydrogen gas detection tests were carried out in the same manner as in Experimental Example 1. However, in this experimental example, resistance sharing voltage measurement was carried out by adopting the circuit diagram illustrated in FIG. 2. The operating temperature T was set to room temperature 24°C (297 K). The applied voltage V was set to 1 V. The measurement was carried out at atmospheric pressure, with a carrier gas on two conditions of $N_2$ gas (flow rate: 500 sccm) and dry air (flow rate: 500 sccm) introduced into a measurement chamber, with a hydrogen gas concentration of 10000 ppm (1%) when hydrogen gas was ON. The hydrogen gas was switched ON and OFF four times. In other words, on the condition of dry air, the hydrogen gas was switched ON at 100 seconds, OFF at 400 seconds, ON at 1000 seconds, OFF at 1300 seconds, ON at 1900 seconds, OFF at 2200 seconds, ON at 2800 seconds, and OFF at 3100 seconds, and the measurement was carried out until 3700 seconds. On the condition of nitrogen, the hydrogen gas was switched ON at 150 seconds, OFF at 400 seconds, ON at 1000 seconds, OFF at 1300 seconds, ON at 1900 seconds, OFF at 2200 seconds, ON at 2800 seconds, and OFF at 3180 seconds, and the measurement was carried out until 3700 seconds.

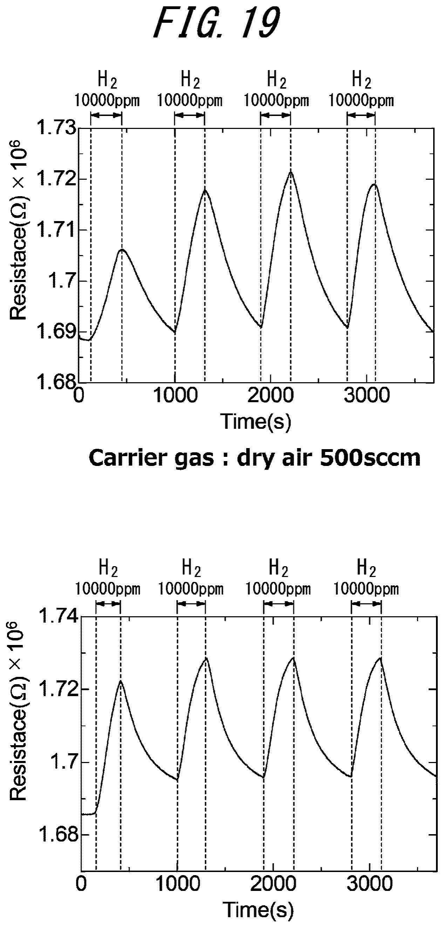

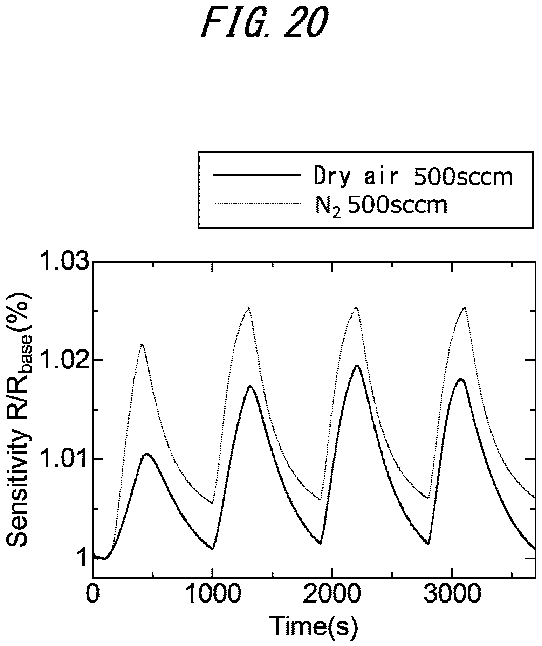

FIG. 19 is a graph illustrating variations in a resistance value over time when the carrier gas is dry air (upper graph) and when the carrier gas is nitrogen (lower graph). The resistance value on the vertical axis is the resistance value of the hydrogen gas sensor, which has been calculated from a measurement value of voltage applied to a resistance portion. FIG. 20 is a graph illustrating variations in a resistance change rate over time when the carrier gas is dry air and when the carrier gas is nitrogen. Here, with respect to $R/R_{base}$ on the vertical axis, $R_{base}$ is a resistance value at 100 seconds (immediately before the hydrogen gas is first ON), and R is a resistance value at a certain time. It was seen from FIGS. 19 and 20 that the sufficient sensitivity (sensitivity based on the resistance change rate) could be obtained regardless of whether the carrier gas was dry air or nitrogen.

[Experimental Example 7: Effect of Wire Thickness D]

Hydrogen gas sensors were produced in the same manner as in Experimental Example 1, with a linewidth W of 80 nm, on two conditions of a wire thickness D of 10 nm and 30 nm, with a wire length L of 7 mm. In this experimental example, a nanowire was arranged as illustrated in FIG. 4 because the wire length L was long.

Hydrogen gas detection tests were carried out in the same manner as in Experimental Example 1. However, in this experimental example, resistance sharing voltage measurement was carried out by adopting the circuit diagram illustrated in FIG. 2. The operating temperature T was set to room temperature 24°C (297 K). The applied voltage V was set to 1 V. The measurement was carried out at atmospheric pressure, with $N_2$ gas (flow rate: 1 SLM), as a carrier gas, introduced into a measurement chamber, with a hydrogen gas concentration of 6000 ppm (0.6%) when hydrogen gas was ON. The hydrogen gas was switched ON and OFF four times. In other words, the hydrogen gas was switched ON at 100 seconds, OFF at 400 seconds, ON at 1000 seconds, OFF at 1300 seconds, ON at 1900 seconds, OFF at 2200 seconds, ON at 2800 seconds, and OFF at 3100 seconds, and the measurement was carried out until 3700 seconds.

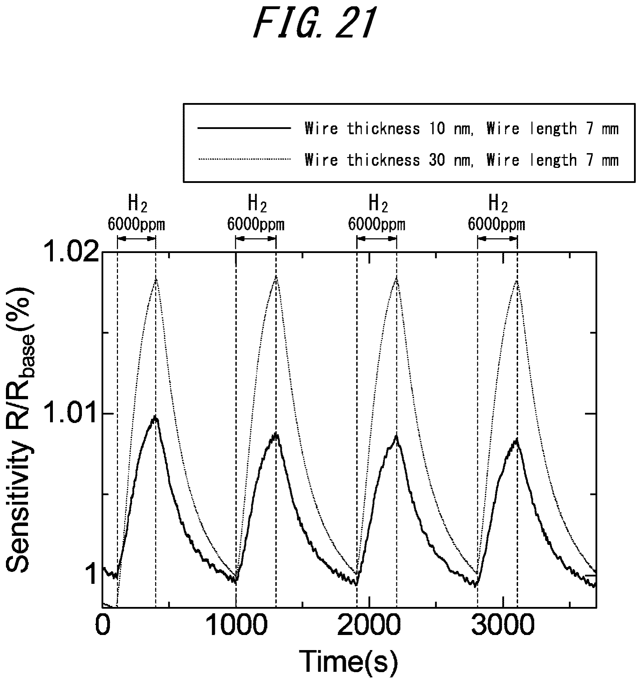

FIG. 21 is a graph illustrating variations in a resistance change rate over time for various wire thicknesses. Here, with respect to $R/R_{base}$ on the vertical axis, $R_{base}$ is a resistance value at 100 seconds (immediately before the hydrogen gas is first ON), and R is a resistance value at a certain time. The resistance value of the hydrogen gas sensor was converted from a measurement value of voltage applied to a resistance portion. It was seen from FIG. 21 that the sufficient sensitivity (sensitivity based on the resistance change rate) could be obtained even with a wire thickness of 10 nm, and that even higher sensitivity was obtained with a wire thickness of 30 nm.

[Experimental Example 8: Repeated Introduction of Hydrogen Gas]

Hydrogen gas sensors were produced in the same manner as in Experimental Example 1, with a linewidth W of 80 nm, on two conditions of "a wire thickness D: 30 nm/ a wire length L: 31 mm" and "a wire thickness D: 10 nm/ a wire length L: 18 mm." In this experimental example, nanowires were arranged as illustrated in FIG. 4 because the wire length L was long.

Hydrogen gas detection tests were carried out in the same manner as in Experimental Example 1. However, in this experimental example, resistance sharing voltage measurement was carried out by adopting the circuit diagram illustrated in FIG. 2. The operating temperature T was set to 100°C (373 K). The applied voltage V was set to 1 V. The measurement was carried out at atmospheric pressure, with $N_2$ gas (flow rate: 10 SLM), as a carrier gas, introduced into a measurement chamber. The hydrogen gas was switched ON and OFF four times. In other words, the hydrogen gas was switched ON at 300 seconds, OFF at 600 seconds, ON at 900 seconds, OFF at 1200 seconds, ON at 1500 seconds, OFF at 1800 seconds, ON at 2100 seconds, and OFF at 2400 seconds, and the measurement was carried out until 3300 seconds. The hydrogen gas concentration when hydrogen gas was ON was set to 30 ppm for the first time, 150 ppm for the second time, 750 ppm for the third time, 1500 ppm for the fourth time.

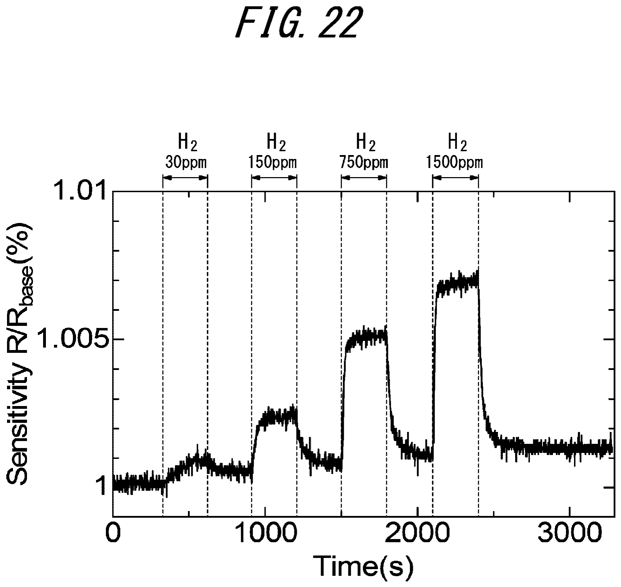

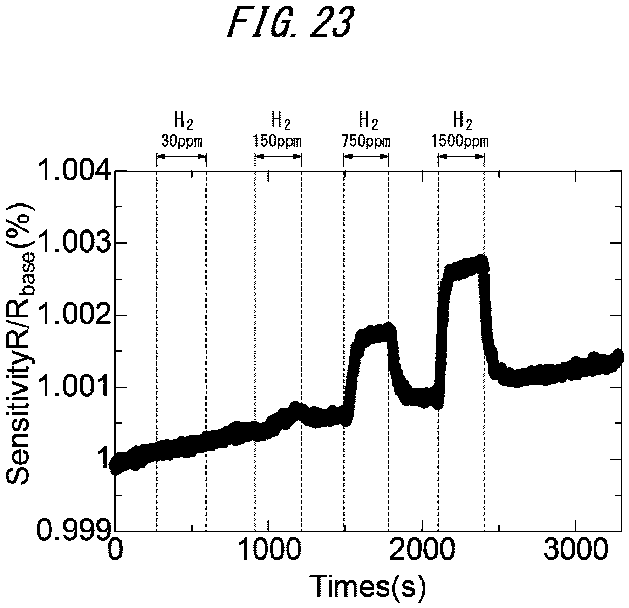

FIG. 22 is a graph illustrating variations in a resistance change rate over time for the repeated introduction of the hydrogen gas for a wire thickness of 30 nm and a wire length of 31 mm. FIG. 23 is a graph illustrating variations in a resistance change rate over time for the repeated introduction of the hydrogen gas for a wire thickness of 10 nm and a wire length of 18 mm. Here, with respect to $R/R_{base}$ on the vertical axis, $R_{base}$ is a resistance value at 300 seconds (immediately before the hydrogen gas is first ON), and R is a resistance value at a certain time. The resistance value of the hydrogen gas sensor was converted from a measurement value of voltage applied to a resistance portion. From FIGS. 22 and 23, it was possible to obtain variations in sensitivity that depended on the hydrogen concentration. The hydrogen gas sensor illustrated in FIG. 22 could detect the hydrogen gas with a concentration of 30 ppm, at an operating temperature of 373 K and an applied voltage of 1 V.

[Experimental Example 9: Effect of Heat Treatment on Nanowire]

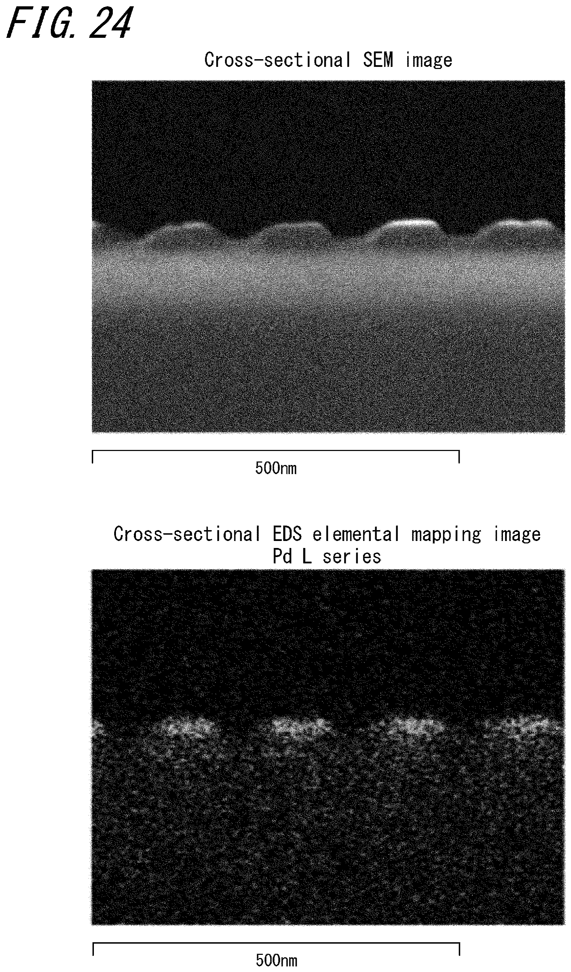

A hydrogen gas sensor was produced in the same manner as in Experimental Example 1, with a linewidth W of 80 nm, a wire thickness D of 30 nm, and a wire length L of 7 mm. In this experimental example, a nanowire was arranged as illustrated in FIG. 4 because the wire length L was long. Note that, after a lift-off process, the nanowire was subjected to heat treatment. The heat treatment was carried out using an infrared lamp annealing device (mini lamp annealer MILA-5000 produced by Advance Riko Inc.), under atmospheric pressure, with a flow of an Ar/H$_2$ (3 volume%) mixed gas, at a heat treatment temperature of 250°C, with holding time of 5 minutes. FIG. 24 illustrates a cross-sectional SEM image (upper image) of the nanowire perpendicular to its extension direction, and an elemental mapping (lower image) of the same area as the cross-sectional SEM image by energy dispersive X-ray spectroscopy (EDS). Since the nanowire is arranged in a zigzag pattern as illustrated in FIG. 4, cross-sections of the nanowire are present periodically. The EDS elemental mapping indicates the intensity of the L-line of Pd, and the bright portions are palladium. The nanowire is in the shape of a dome in cross-section, which is constituted of curved both end portions and a flat portion. In the corresponding SEM image, the radius of curvature increases due to the heat treatment, as compared to FIG. 6. The ratio in area of portions enclosed by curved surfaces increases, so it is thought that the ratio of a cross-sectional area to which internal stress is applied increases, compared to before the heat treatment.

Hydrogen gas detection tests were carried out in the same manner as in Experimental Example 1. However, in this experimental example, resistance sharing voltage measurement was carried out by adopting the circuit diagram illustrated in FIG. 2. The operating temperature T was set on four conditions of 24°C, 50°C, 100°C, and 150°C. The applied voltage V was set to 1 V. The measurement was carried out at atmospheric pressure, with N$_2$ gas (flow rate: 1 SLM), as a carrier gas, introduced into a measurement chamber, with a hydrogen gas concentration of 3333 ppm (0.33%) when hydrogen gas was ON. The hydrogen gas was switched ON and OFF four times. In other words, the hydrogen gas was switched ON at 100 seconds, OFF at 400 seconds, ON at 1000 seconds, OFF at 1300 seconds, ON at 1900 seconds, OFF at 2200 seconds, ON at 2800 seconds, and OFF at 3100 seconds, and the measurement was carried out until 3700 seconds. After the hydrogen gas detection tests were carried out on the above four conditions for the operating temperature, a similar hydrogen gas detection test was carried out again at an operating temperature of 21°C (room temperature).

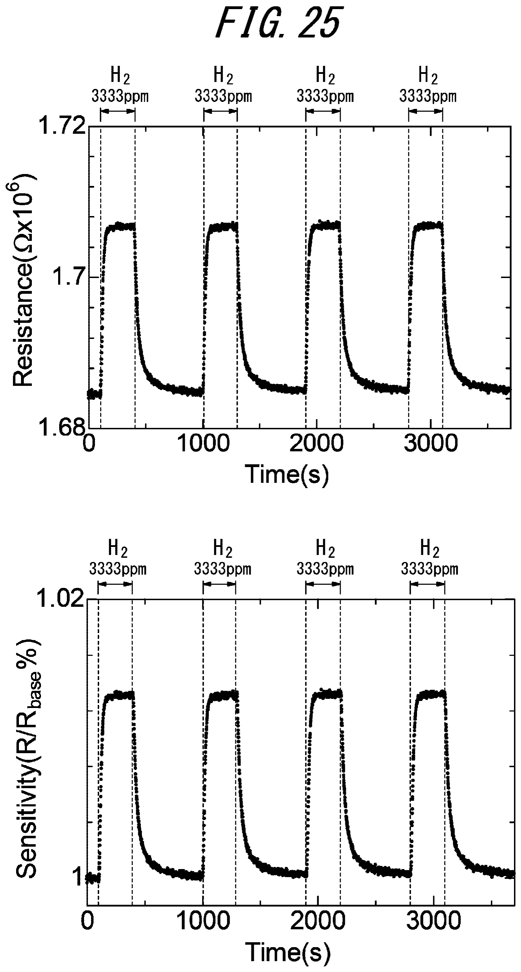

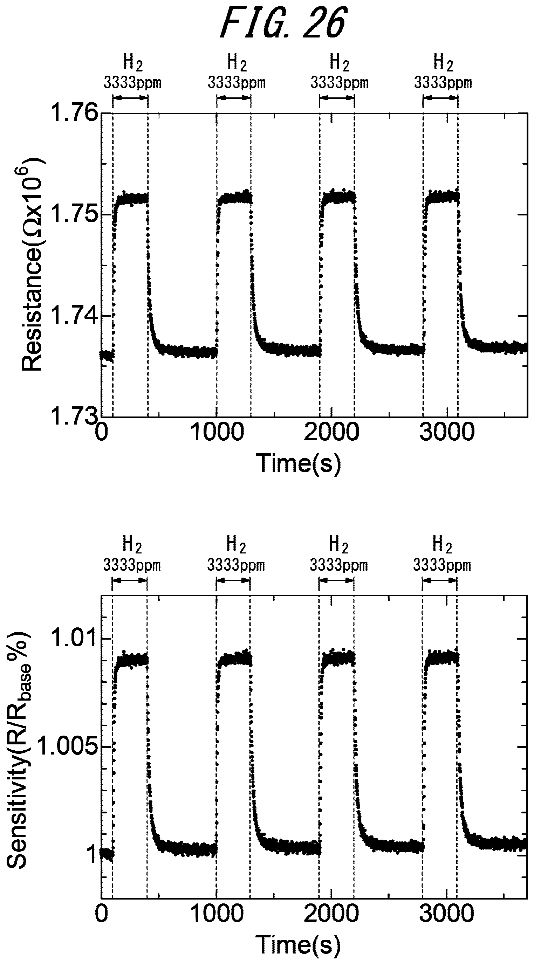

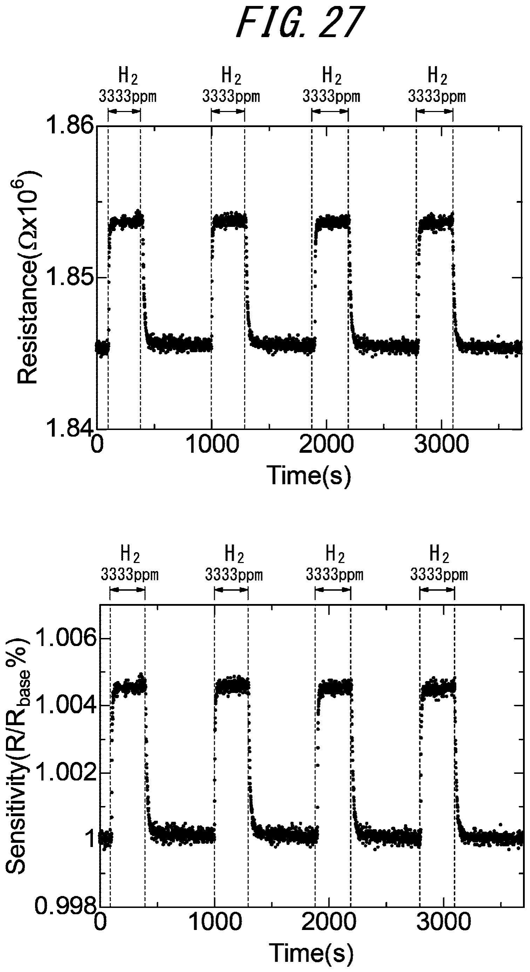

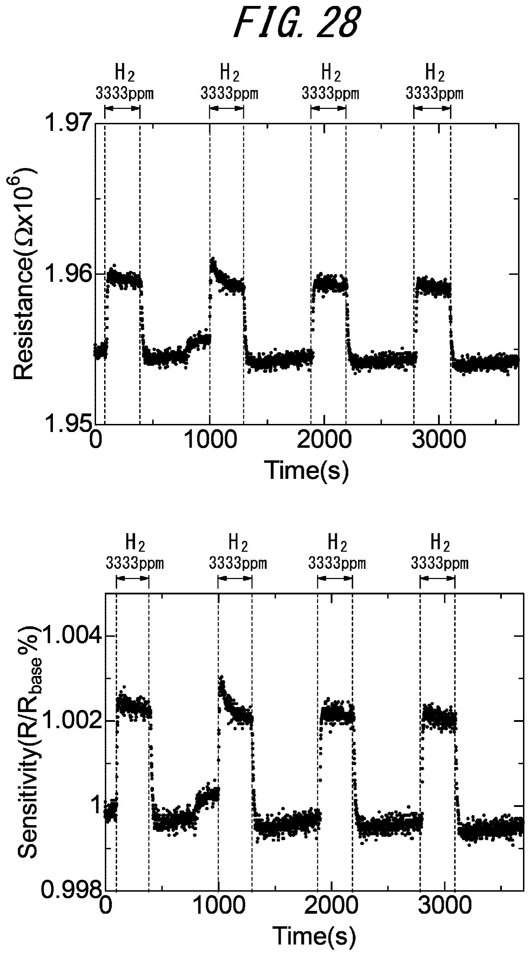

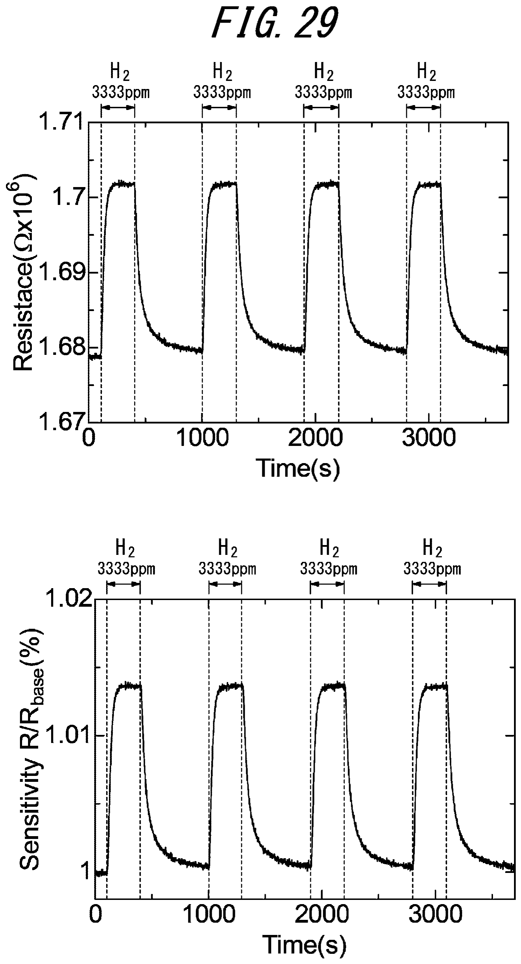

FIGS. 25 to 29 are (upper) graphs illustrating variations in a resistance value over time and (lower) graphs illustrating variations in a resistance change rate over time, at each operating temperature. The resistance value on the vertical axis of the (upper) graphs is the resistance value of the hydrogen gas sensor, which has been calculated from a measurement value of voltage applied to a resistance portion. With respect to R/R$_{base}$ on the vertical axis of the (lower) graphs, R$_{base}$ is a resistance value at 100 seconds (immediately before the hydrogen gas is first ON), and R is a resistance value at a certain time.

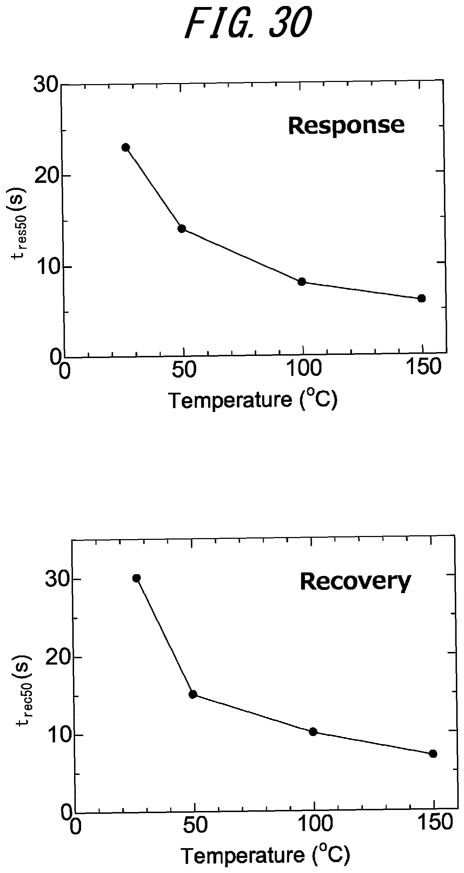

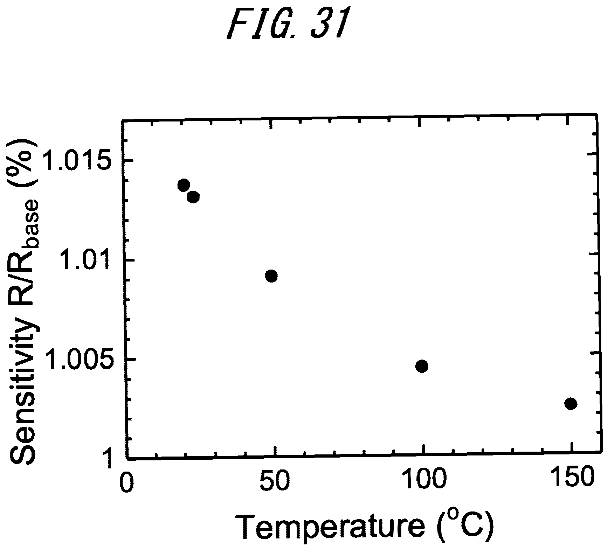

FIG. 30 is a (upper) graph illustrating the relationship between operating temperature and response time t$_{res50}$ and a (lower) graph illustrating the relationship between operating temperature and recovery time t$_{rec50}$. The vertical axis of the (upper) graph is the average value of t$_{res50}$ at four ON/OFF times. The vertical axis of the (lower) graph is the average value of t$_{rec50}$ at four ON/OFF times. FIG. 31 is a graph illustrating the relationship between operating temperature and sensitivity (sensitivity based on the resistance change rate). The sensitivity R/R$_{base}$ on the vertical axis of FIG. 31 is the average value of R/R$_{base}$ at four OFF times (400 seconds, 1300 seconds, 2200 seconds, and 3100 seconds) when R$_{base}$ is fixed as a resistance value at 100 seconds.

Referring to FIG. 30, the hydrogen gas sensor that was subjected to the heat treatment had better response/recovery characteristics than the hydrogen gas sensors that were not subjected to the heat treatment (FIGS. 9 and 10). When the heat treatment was performed, both the response time t$_{res50}$ and the recovery time t$_{rec50}$ were shortened at each operating temperature, thus enabling high-speed response and high-speed recovery. Referring to FIG. 31, the sensitivity deteriorated as the operating temperature increased. This trend is consistent with the trend in FIG. 8.

The reason why the response/recovery time is shorter when the heat treatment is performed than when the heat treatment is not performed is thought to be that, although the radius of curvature increases due to the heat treatment, the ratio of portions enclosed by curved surfaces increases, so the ratio of a cross-sectional area to which internal stress is applied increases compared to when the linewidth is wider, and a region in which internal stress increases due to the radius of curvature is more optimized.

[Experimental Example 10: Effect of Anneal Treatment on Nanowire]

Hydrogen gas sensors were produced in the same manner as in Experimental Example 1, with a linewidth W of 55 nm, a wire thickness D of 30 nm, and a wire length L of 0.7 mm. In this experimental example, nanowires were arranged as illustrated in FIG. 4 because the wire length L was long. Note that, after a lift-off process, the nanowires were subjected to an exposure step and heat treatment under the following conditions.

<Condition 1>

Neither an exposure step nor a heat treatment step was carried out.

<Condition 2>

The nanowire was subjected to an exposure step by supplying an Ar/H$_2$ (3 volume%) mixed gas for 3 minutes. After that, heat treatment was carried out using an infrared lamp annealing device (mini lamp annealer MILA-5000 produced by Advance Riko Inc.), in an atmosphere of an Ar/H$_2$ (3 volume%) mixed gas at atmospheric pressure, under the conditions of heat treatment temperature: 250°C, temperature increase rate: 10°C/min, and holding time: 5 min.

<Conditions 3 to 5>

The nanowires were subjected to an exposure step by supplying an Ar/H$_2$ (3 volume%) mixed gas for 3 minutes. After that, RTA treatment was carried out using an RTA device (MILA-5000UHV produced by Advance Riko Inc.), in an atmosphere of an Ar/H$_2$ (3 volume%) mixed gas, at atmospheric pressure, under the conditions of heat treatment temperature: 500°C (Condition 3), 400°C (Condition 4), 600°C (Condition 5), temperature increase rate: 50°C/sec, and holding time: 0 min.

<Crystal State of Palladium Constituting Nanowire>

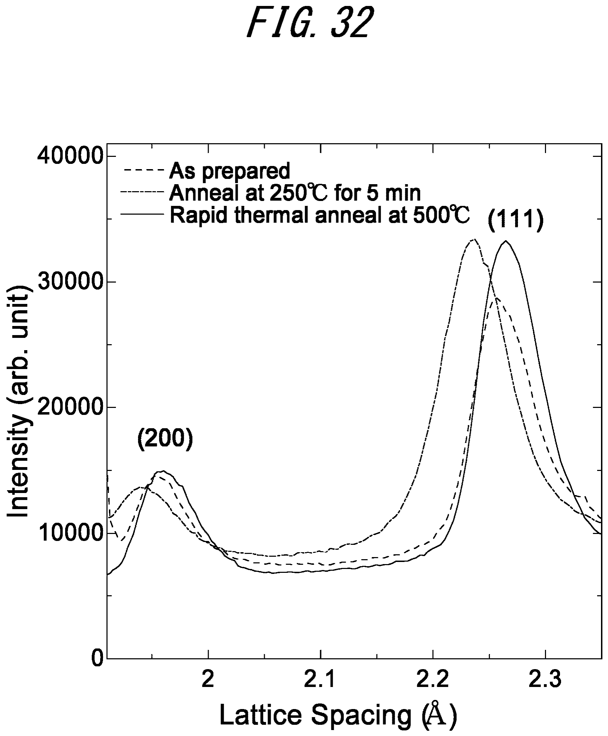

The samples on Conditions 1 to 3 were subjected to GI-WAXS measurement using SPring-8 beamline BL13XU. The X-ray energy was set to 12.39797043082328 eV. FIG. 32 illustrates X-ray diffraction spectra obtained for the respective samples. As can be seen in FIG. 32, the intensity and area of the peak attributed to Pd (111) were significantly larger in Conditions 2 and 3, which were subjected to the exposure step and the heat treatment step, than in Condition 1, which was not subjected to the exposure step and the heat treatment step. This indicates that palladium constituting the nanowire has been polycrystallized in Conditions 2 and 3.

The lattice constant of palladium calculated from the peak attributed to Pd (111) and the peak attributed to Pd (200) was 3.909 angstroms for Condition 1, 3.879 angstroms for Condition 2, and 3.923 angstroms for Condition 3. The lattice constant of the $\alpha$-phase of palladium is 3.90 $\pm$ 0.02 angstroms, so it can be said that all Conditions 1 to 3 are in the $\alpha$-phase, but in Condition 3, which was subjected to the RTA treatment with palladium occluded hydrogen, the lattice expansion state was maintained, and the lattice constant was close to the upper limit of the lattice constant of the $\alpha$-phase.

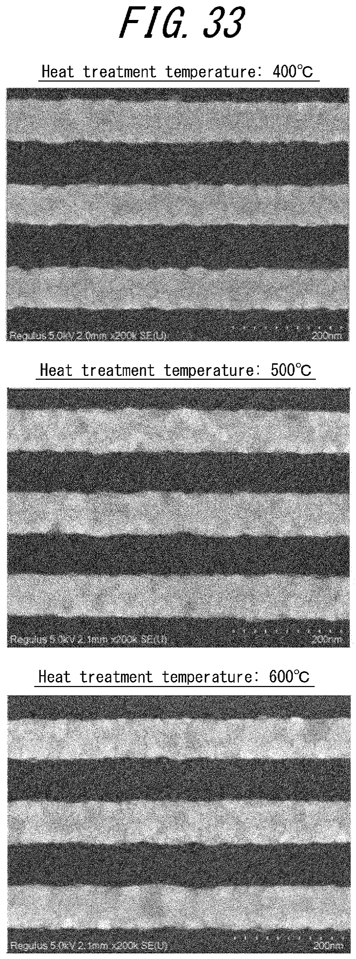

FIG. 33 illustrates SEM images of top surfaces of the nanowires in the samples for Conditions 3 to 5. As illustrated in FIG. 33, in Conditions 3 to 5, which were subjected to the RTA treatment after the exposure step, many grains of palladium can be seen in the nanowires with a uniform linewidth (approximately 80 nm), which indicates that palladium has been polycrystallized.

<Hydrogen Gas Detection Test 1>

Hydrogen gas detection tests were carried out in the same manner as in Experimental Example 1. The operating temperature T was set to room temperature 21°C (294 K). The applied voltage V was set to 1 V. The measurement was carried out at atmospheric pressure, with N$_2$ gas (flow rate: 3 SLM), as a carrier gas, introduced into a measurement chamber. The hydrogen gas was switched ON and OFF three times. In other words, the hydrogen gas was switched ON at 100 seconds, OFF at 250 seconds, ON at 400 seconds, OFF at 550 seconds, ON at 700 seconds, and OFF at 850 seconds, and the measurement was carried out until 1050 seconds. The hydrogen concentration when the hydrogen gas was ON was set to 500 ppm for the first time, 1000 ppm for the second time, and 5000 ppm for the third time.

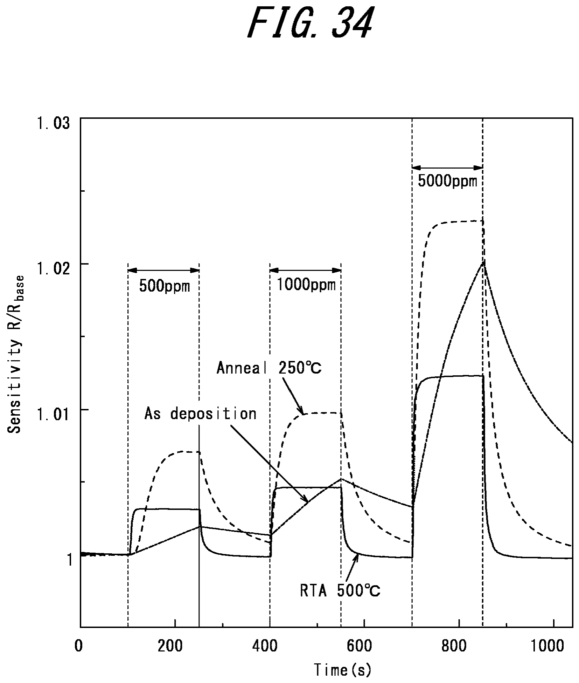

FIG. 34 is a graph illustrating variations in a resistance change rate over time in Conditions 1 to 3. Here, with respect to R/R$_{base}$ on the vertical axis, R$_{base}$ is a resistance value at 100 seconds (immediately before the hydrogen gas is first ON), and R is a resistance value at a certain time. It was seen from FIG. 34 that Conditions 2 and 3, which were subjected to the exposure step and the heat treatment step, could achieve high-speed response and high-speed recovery than Condition 1 (As deposition), which was not subjected to the exposure step and the heat treatment step. In particular, Condition 3, which was subjected to the RTA treatment after the exposure step, achieved extremely high-speed response and recovery, so the response/recovery characteristics were significantly improved.

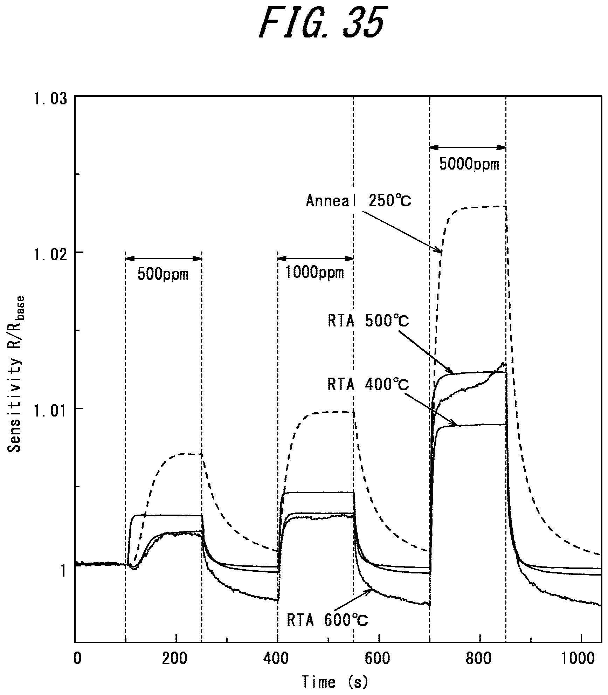

FIG. 35 is a graph illustrating variations in a resistance change rate over time in Conditions 2 to 5. It was seen from FIG. 35 that all Conditions 3 to 5, in which the heat treatment temperature for the RTA treatment was set to 400°C, 500°C, and 600°C, respectively, could achieve high-speed response and high-speed recovery than Condition 2, but Condition 3, in which the heat treatment temperature was set to 500°C, had the best response/recovery characteristics.

<Hydrogen Gas Detection Test 2>

A hydrogen gas detection test was carried out using the hydrogen gas sensor on Condition 3, in the same manner as in Experimental Example 1. The operating temperature T was set to room temperature 21°C (294 K). The applied voltage V was set to 1 V. The measurement was carried out at atmospheric pressure, with N$_2$ gas (flow rate: 3 SLM), as a carrier gas, introduced into a measurement chamber. The hydrogen gas was switched ON and OFF five times. In other words, the hydrogen gas was switched ON at 100 seconds, OFF at 250 seconds, ON at 400 seconds, OFF at 550 seconds, ON at 700 seconds, OFF at 850 seconds, ON at 1000 seconds, OFF at 1150 seconds, ON at 1300 seconds, and OFF at 1450 seconds, and the measurement was carried out until 1600 seconds. The hydrogen concentration when the hydrogen gas was ON was set to 297 ppm for the first time, 2727 ppm for the second time, 4286 ppm for the third time, 10000 ppm for the fourth time, and 30000 ppm for the fifth time.

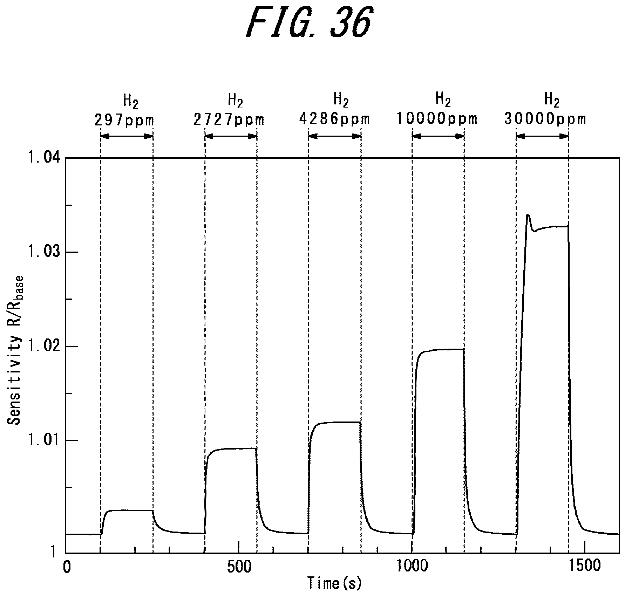

FIG. 36 is a graph illustrating variations in a resistance change rate over time. Here, with respect to R/R$_{base}$ on the vertical axis, R$_{base}$ is a resistance value at 100 seconds (immediately before the hydrogen gas is first ON), and R is a resistance value at a certain time. It was seen from FIG. 36 that the hydrogen gas sensor on Condition 3 could detect hydrogen gas with an extremely low concentration, i.e., 297 ppm at high speed, and the recovery was also fast.

INDUSTRIAL APPLICABILITY

The hydrogen gas sensor according to this disclosure has high sensitivity and excellent response/recovery characteristics, and can detect hydrogen gas with low power consumption, and therefore may be applicable to mobile gas sensors and other applications.

REFERENCE SIGNS LIST

| | |
|---|---|
| 100 | hydrogen gas sensor |
| 10 | substrate |
| 12A | first pad electrode |
| 12B | second pad electrode |
| 14 | nanowire |
| 14A | flat portion of nanowire |
| 14B1 | curved surface portion of nanowire |
| 14B2 | curved surface portion of nanowire |
| 18 | power supply |
| 20 | ammeter |
| 22 | voltmeter |
| 24 | resistance |
| 30 | resist film |
| 32 | mask pattern |
| 34 | metal film |
| 34A | first portion of metal film |
| 34B | second portion of metal film |
| W | linewidth of nanowire |
| D | thickness of nanowire |
| L | length of nanowire |

**Claims**

1. A hydrogen gas sensor comprising:

   a substrate having an insulating surface;
   a first pad electrode and a second pad electrode that are formed on the insulating surface of the substrate; and
   a nanowire made of a hydrogen storage metal, the nanowire being formed on the insulating surface of the substrate to connect the first pad electrode and the second pad electrode, the nanowire having a linewidth of 50 nm or more and 150 nm or less, and a thickness of 10 nm or more and 60 nm or less,
   wherein the hydrogen gas sensor is configured to detect hydrogen gas based on a variation in an electric signal detected between the first pad electrode and the second pad electrode by passing a current between the first pad electrode and the second pad electrode.

2. The hydrogen gas sensor according to claim 1, wherein the nanowire has a linewidth of 80 nm or more and 100 nm or less.

3. The hydrogen gas sensor according to claim 1, wherein the nanowire has a thickness of 20 nm or more and 50 nm or less.

4. The hydrogen gas sensor according to claim 1, wherein the nanowire has a length of 10 $\mu$m or more and 300 mm or less.

5. The hydrogen gas sensor according to claim 4, wherein the nanowire has a length of 0.07 mm or more.

6. The hydrogen gas sensor according to claim 5, wherein the nanowire has a length of 0.5 mm or more.

7. The hydrogen gas sensor according to any one of claims 1 to 6, wherein the hydrogen storage metal is one or more selected from

(I) single elements of palladium (Pd), rhodium (Rh), iridium (Ir), ruthenium (Ru), osmium (Os), vanadium (V), titanium (Ti), zirconium (Zr), lanthanum (La), tungsten (W), calcium (Ca), magnesium (Mg), strontium (Sr), barium (Ba), and beryllium (Be), as exothermic metals A that easily form stable hydrides, and solid solution alloys of these exothermic alloys A; and
(II) $AB_5$-type alloys, $AB_2$-type alloys, $AB$-type alloys, and $A_2B$-type alloys that are combinations of the exothermic metal(s) A and one or more selected from nickel (Ni), iron (Fe), cobalt (Co), manganese (Mn), and zinc (Zn), as endothermic metals B that have no affinity for hydrogen.

8. The hydrogen gas sensor according to any one of claims 1 to 6, wherein the hydrogen storage alloy is palladium (Pd).

9. The hydrogen gas sensor according to claim 8, wherein the palladium constituting the nanowire is polycrystallized.

10. The hydrogen gas sensor according to claim 9, wherein the palladium constituting the nanowire has a lattice constant of 3.925 $\pm$ 0.005 angstroms.

11. A method of producing a hydrogen gas sensor, the method comprising:

preparing a substrate having an insulating surface;
forming a first pad electrode and a second pad electrode on the insulating surface of the substrate; and
forming a nanowire made of a hydrogen storage metal on the insulating surface of the substrate to connect the first pad electrode and the second pad electrode, the nanowire having a linewidth of 50 nm or more and 150 nm or less, and a thickness of 10 nm or more and 60 nm or less,
wherein the method produces a hydrogen gas sensor that is configured to detect hydrogen gas based on a variation in an electric signal detected between the first pad electrode and the second pad electrode by passing a current between the first pad electrode and the second pad electrode.

12. The method of producing a hydrogen gas sensor according to claim 11, the method comprising:

exposing the nanowire to an atmosphere containing hydrogen and an inert gas; and
thereafter subjecting the nanowire to heat treatment under an atmosphere containing hydrogen and an inert gas.

13. The method of producing a hydrogen gas sensor according to claim 12, wherein the heat treatment is RTA treatment that is carried out at a heat treatment temperature of 350°C or more and 650°C or less.

14. The method of producing a hydrogen gas sensor according to any one of claims 11 to 13, wherein the hydrogen storage alloy is palladium (Pd).

# FIG. 1A

# FIG. 1B

## FIG. 1C

## FIG. 1D

*FIG. 2*

*FIG. 3*

*FIG. 4*

EP 4 671 751 A1

*FIG. 5A*

*FIG. 5B*

*FIG. 5C*

*FIG. 5D*

# FIG. 6

Linewidth 40 nm

Linewidth 50 nm

Linewidth 80 nm

Linewidth 100 nm

Linewidth 200 nm

FIG. 7

$T = 50°C$

# FIG. 8

## FIG. 9

# FIG. 10

Legend:
- ○ T = 50°C
- △ T = 60°C
- □ T = 70°C
- × T = 80°C
- ◇ T = 90°C
- + T = 100°C
- ● T = 110°C

Y-axis: $t_{rec50}$ (s)

X-axis: Linewidth (nm)

Recovery

# FIG. 11

# FIG. 12

# FIG. 13A

Wire length 0.7mm

Wire length 7mm

# FIG. 13B

Wire length 14mm

Wire length 21mm

## FIG. 14

## FIG. 15

Response

Recovery

## FIG. 16

# FIG. 17

## FIG. 18

## FIG. 19

Carrier gas : dry air 500sccm

# FIG. 20

# FIG. 21

## FIG. 22

# FIG. 23

*FIG. 24*

Cross-sectional SEM image

500nm

Cross-sectional EDS elemental mapping image
Pd L series

500nm

## FIG. 25

## FIG. 26

# FIG. 27

# FIG. 28

## FIG. 29

# FIG. 30

Response

Recovery

## FIG. 31

# FIG. 32

# FIG. 33

Heat treatment temperature: 400°C

Heat treatment temperature: 500°C

Heat treatment temperature: 600°C

# FIG. 34

# FIG. 35

## FIG. 36

## INTERNATIONAL SEARCH REPORT

| International application No. |
|---|
| **PCT/JP2024/006393** |

| A. | CLASSIFICATION OF SUBJECT MATTER |
|---|---|

*G01N 27/12*(2006.01)i
FI:   G01N27/12 B; G01N27/12 C

According to International Patent Classification (IPC) or to both national classification and IPC

| B. | FIELDS SEARCHED |
|---|---|

Minimum documentation searched (classification system followed by classification symbols)

G01N27/00-27/24

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2005-537469 A (NANO PROPRIETARY INC.) 08 December 2005 (2005-12-08) claims, paragraph [0022], fig. 3 | 1-6, 8, 11, 14 |
| A | | 7, 9-10, 12-13 |
| X | US 2008/0106276 A1 (THE REGENTS OF THE UNIVERSITY OF CALIFORNIA) 08 May 2008 (2008-05-08) paragraphs [0033]-[0038], fig. 1-3 | 1-6, 8-11, 14 |
| A | | 7, 12-13 |
| A | CN 111487289 A (NANJING UNIVERSITY) 04 August 2020 (2020-08-04) entire text, all drawings | 1-14 |
| A | CN 106018490 A (HUBEI UNIVERSITY) 12 October 2016 (2016-10-12) entire text, all drawings | 1-14 |
| A | US 2010/0005853 A1 (NANO-PROPRIETARY, INC.) 14 January 2010 (2010-01-14) entire text, all drawings | 1-14 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 May 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2024/006393**

### C. DOCUMENTS CONSIDERED TO BE RELEVANT

| Category* | Citation of document, with indication, where appropriate, of the relevant passages | Relevant to claim No. |
|---|---|---|
| A | JP 2018-138876 A (KABUSHIKI KAISHA TOSHIBA) 06 September 2018 (2018-09-06)<br>entire text, all drawings | 1-14 |
| A | JP 2018-071975 A (KABUSHIKI KAISHA TOSHIBA) 10 May 2018 (2018-05-10)<br>entire text, all drawings | 1-14 |
| A | JP 2009-025229 A (SEIKO INSTRUMENTS INC.) 05 February 2009 (2009-02-05)<br>entire text, all drawings | 1-14 |

## INTERNATIONAL SEARCH REPORT
### Information on patent family members

International application No.

**PCT/JP2024/006393**

| Patent document cited in search report | | | Publication date (day/month/year) | Patent family member(s) | | | Publication date (day/month/year) |
|---|---|---|---|---|---|---|---|
| JP | 2005-537469 | A | 08 December 2005 | US | 2004/0070006 | A1 | |
| | | | | claims, paragraph [0029], fig. 3 | | | |
| | | | | US | 2005/0005675 | A1 | |
| | | | | US | 2005/0155858 | A1 | |
| | | | | US | 2008/0078234 | A1 | |
| | | | | WO | 2004/020978 | A2 | |
| | | | | WO | 2006/091553 | A2 | |
| | | | | KR | 10-2005-0071483 | A | |
| | | | | CA | 2596129 | A1 | |
| | | | | KR | 10-2007-0113223 | A | |
| | | | | CN | 101124476 | A | |
| US | 2008/0106276 | A1 | 08 May 2008 | US | 2003/0079999 | A1 | |
| | | | | US | 6843902 | B1 | |
| | | | | US | 2004/0238367 | A1 | |
| | | | | US | 2008/0128284 | A1 | |
| | | | | WO | 2003/008954 | A1 | |
| CN | 111487289 | A | 04 August 2020 | (Family: none) | | | |
| CN | 106018490 | A | 12 October 2016 | (Family: none) | | | |
| US | 2010/0005853 | A1 | 14 January 2010 | JP | 2009-534670 | A | |
| | | | | US | 2007/0125153 | A1 | |
| | | | | US | 2007/0240491 | A1 | |
| | | | | WO | 2007/019244 | A2 | |
| | | | | WO | 2007/124408 | A2 | |
| | | | | CA | 2615107 | A1 | |
| | | | | KR | 10-2008-0036627 | A | |
| | | | | CN | 101501480 | A | |
| | | | | CA | 2649557 | A1 | |
| | | | | KR | 10-2009-0007443 | A | |
| | | | | CN | 101467030 | A | |
| JP | 2018-138876 | A | 06 September 2018 | (Family: none) | | | |
| JP | 2018-071975 | A | 10 May 2018 | (Family: none) | | | |
| JP | 2009-025229 | A | 05 February 2009 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. XU** ; **M. P. ZACH**. Self-assembled monolayer-enhanced hydrogen sensing with ultrathin palladium films. *Appl. Phys. Lett.*, 2005, vol. 86, 203104